# EUROPEAN PATENT SPECIFICATION

(11) **EP 3 484 462 B1**
(45) Date of publication and mention of the grant of the patent: **14.12.2022**
(21) Application number: 17742997.4
(22) Date of filing: 13.07.2017
(51) Int. Cl.: A61K 31/138, A61K 31/495, A61P 17/02, A61K 8/49

(54) **5-HYDROXYTRYPTAMINE 1B RECEPTOR-STIMULATING AGENT FOR SKIN AND/OR HAIR REPAIR**
5-HYDROXYTRYPTAMIN-1B-REZEPTORSTIMULIERENDES MITTEL ZUR HAUT- UND/ODER HAARREPARATUR
AGENT STIMULANT LE RÉCEPTEUR DE LA 5-HYDROXYTRYPTAMINE 1B POUR LA RÉPARATION DE LA PEAU ET/OU DES CHEVEUX.

(30) Priority: 15.07.2016 WO PCT/IB2016/001136
(43) Date of publication of application: 22.05.2019
(73) Proprietor: Institut Pasteur, 75015 Paris (FR); Groupe Hospitalier Universitaire Paris - Psychiatrie et Neurosciences, 75014 Paris (FR); Université Paris Cité, 75006 Paris (FR)
(72) Inventor: GAILLARD, Raphael, 75013 Paris (FR); CHRETIEN, Fabrice, Bruno, 75008 Paris (FR); ROCHETEAU, Pierre, 75014 Paris (FR); TORNO, Nicolas, 75724 Paris Cedex 15 (FR)
(74) Representative: Regimbeau
(86) International application number: PCT/EP2017/067791
(87) International publication number: WO 2018/011382

(56) References cited:
- WO-A1-03/029232
- WO-A1-2008/078353
- WO-A1-2011/048496
- WO-A1-2014/190063
- WO-A2-98/31335
- WO-A2-2007/148341
- ALEX MALININ ET AL: "Treatment with selective serotonin reuptake inhibitors for enhancing wound healing", MEDICAL HYPOTHESES., vol. 63, no. 1, 1 January 2004 (2004-01-01), pages 103-109, XP055348687, US ISSN: 0306-9877, DOI: 10.1016/j.mehy.2003.10.021
- Nguyen: "753 Serotonin as a potential therapeutic target for mesenchymal stem cell - Mediated improvement of wound healing", Journal of Investigative Dermatology, S133 Published in issue: May 2016, 1 May 2016 (2016-05-01), XP055348757, Retrieved from the Internet: URL:http://www.jidonline.org/article/S0022 -202X(16)30850-8/pdf [retrieved on 2017-02-22]

## Description

### INTRODUCTION

The present invention is defined in the appended claims. It relates to the field of skin and/or hair repair, for therapeutic and cosmetic purposes. It more specifically relates to an agent stimulating the 5-hydroxytryptamine 1B receptor, for use for improving or boosting skin and/or hair repair.

Traumatic injury is the leading cause of mortality in Europe and the United States and in addition millions of surgical wounds are created annually in the course of routine medical care (Sen et al., 2009). Facilitating the healing of these injuries and minimizing the aesthetic impact on the patient and maximal restoration of tissue function remains a central concern in clinical care. Although minor injuries in healthy individuals generally heal well, larger injuries or the presence of a variety of physiological or common disease states including age, infection, diabetes/vascular disease, and cancer can negatively affect the healing process in ways that are currently poorly understood. Moreover, mechanisms underlying pathological scarring, including hypertrophic scarring and more extreme keloid formation, are elusive, and efficient treatment options are currently missing (Reish and Eriksson, 2008).

Demographically, the number of patients suffering from chronic wounds and impaired healing conditions is reaching epidemic proportions and will become even more burdensome in both human health and economic terms (Brem and Tomic-Canic, 2007). It is thus key to find new treatments of non-healing wounds or for speeding up the repair of acute wounds and reducing scar formation.

As the outer barrier, the skin is the organ most challenged by a range of external stress factors, resulting in frequent cell and barrier damage. As such, the skin has developed a set of complex mechanisms to protect itself and to restore tissue integrity when damaged, without resulting in septicemia. Experimentally, because of its accessibility, the skin is one of the best organs in which to study response mechanisms to tissue damage and during repair. Findings from such research in skin have contributed to the unravelling of novel fundamental principles in regenerative biology. In normal physiological conditions, restoration of a functional epidermal barrier is highly efficient, whereas postnatal repair of the deeper dermal layer is less so and results in scar formation with a substantial loss of original tissue structure and function. When the normal repair response does not work properly, there are two major outcomes: either an ulcerative skin defect (chronic wound) or an excessive formation of scar (hypertrophic scar or keloid). This scarification and repeated scarification also occurs in many chronic inflammatory or autoimmune disorders, or even in self-scarification in patients afflicted with particular psychiatric disorders, such as Autism Spectrum Disorders (ASD) ; which upon appropriate treatment can be alleviated or even cured but leaving skin redness, non aesthetic repairs. As fully described hereafter, the present invention provides a solution to accelerate tissue and skin repairs and improve the function as well as improve tissue and skin structure to an extent leading to restoring significantly the original and natural appearance, structure, function or both.

Skin is a very interesting paradigm to study the regenerative processes thanks to its incredible capacity to regenerate and its ease of accessibility and unveiled many fundamental processes principles in regenerative biology. There thus is a need in the art to identify new agents capable of regenerating the skin, not only for therapeutic purpose, i.e. for treating any loss, damage or impairment of the skin such as scares, burns or wounds, but also for cosmetic purpose. The invention as depicted below provides a solution to delay or slow down skin ageing in promoting stem cells to accelerate tissue or skin natural reconstruction or replacement. More specifically the invention moves the balance between natural or induced senescence in favor of tissue or skin replacement, this without observing exhaustion of the reservoir of stem cells.

Besides, an important part of the skin is the hair. Hair growth requires inductive interactions between dermal and epidermal cells and occurs in cycles throughout adult life, providing a uniquely accessible system for studying mammalian signaling pathways. Normal hair growth requires the maintenance of a delicate balance between the proliferation and the differentiation of pluripotent epidermal cells in the bulb region of active hair follicles. Defects in the differentiation of follicular epidermal cells can lead to hair-loss diseases such as alopecia universalis (Ahmad et al., 1998). There thus is a need in the art to identify new agents capable of stimulating hair growth, not only to treat pathological hair loss such as alopecia, but also to promote natural hair growth for cosmetic purpose.

So far, many claims have been made for compounds or plants extracts to promote hair growth which in fact only result in best cases in slowing down hair loss. The most known compounds to have such effect are stemoxydin, aminexil, minoxidin and Finasterin. For example, regarding stemoxydin, it was shown in about 100 patients an average of 1500 new hairs in 90 days which is +4% compared to the normal average of 50 to 100 new hairs growing every day. Therefore, there is still a large room for improvements in results in this technical field. In fact, the endpoint of treatment of scalp and hair is rather preventive to slow down natural and induced hair deeming or loss over time. The present inventors identified a new class of compounds which address this preventive need (alone or in combination with the above molecules, such as stemoxidine), in accelerating stem cells activation.

In regard to the above, the Inventors have indeed observed and demonstrated for the first time that agents stimulating the 5-hydroxytryptamine 1B receptor (namely, herein fluoxetine and vortioxetine), either directly or indirectly, can trigger skin regeneration after inducing skin injury in mice, and also promote hair growth in shaved mice.

These results are very promising and pave the way to the development of new drugs and cosmetics that can promote and/or accelerate skin and/or hair repair.

### DETAILED DESCRIPTION OF THE INVENTION

Unless stated otherwise, scientific and technical terms used in connection with the present invention shall have the meanings that are commonly understood by those of ordinary skill in the art. Furthermore, unless otherwise required by context, nomenclatures used herein, and techniques of molecular biology, cell culture, and pharmacology are those well-known and commonly used in the art. Such techniques are fully explained in the literature (see Ausubel et al., Current Protocols in Molecular Biology, Eds., John Wiley & Sons, Inc. New York, 2013; Remington: The Science and Practice of Pharmacy, 22nd ed., Mack Publishing Co., Easton, Pa., 2012).

Definitions are provided throughout the specification.

According to the different aspects and embodiments of the invention described herein, a "subject" or "host" refers to a subject possessing a serotonergic system, said system comprising more particularly 5-hydroxytryptamine receptors, including notably the 5-hydroxytryptamine 1B receptor. Said subject thus preferably includes animals and humans. Besides, in the context of the present invention, the subject is preferably not suffering from, or is not treated for, a migraine, headache and/or a psychiatric disorder, such as depression, anxiety or bipolar disorder, to name a few.

The present invention may be understood more readily by reference to the following detailed description, including preferred embodiments of the invention, and examples included herein.

### Applications according to the invention

As indicated above, the present inventors have discovered that the administration of vortioxetine or fluoxetine, which are well-known inhibitors of serotonin reuptake, accelerates wound closure of injured mice and increases hair growth after shaving. Interestingly, wound healing and hair growth were greater with vortioxetine than with fluoxetine, when assessed on the same timeframe. The inventors further established that these effects were mediated by these inhibitors via the 5-HT1 BR serotonin receptor.

The present invention is directed to a 5-hydroxytryptamine 1B receptor (5-HT1 BR)-stimulating agent for its therapeutical use as a booster of skin and/or hair repair and to the cosmetic use of a 5-hydroxytryptamine 1B receptor (5-HT1 BR)-stimulating agent for boosting skin and/or hair repair.

More precisely, the disclosure relates to the use of a 5-hydroxytryptamine 1B receptor (5-HT1 BR)-stimulating agent as described herein, for manufacturing a medicament or a cosmetic composition intended to boost skin and/or hair repair. In other words, the disclosure relates to a method for repairing skin and/or hair or for boosting skin and/or hair repair, comprising the step of administering an effective amount of a 5-hydroxytryptamine 1B receptor (5-HT1 BR)-stimulating agent as described herein, to said subject.

By "booster" or "boosting" of skin and/or hair repair, it is meant herein the promotion and/or acceleration of skin and/or hair repair that naturally occurs in a living subject.

By "skin repair" or "skin regeneration", it is meant herein repair or regeneration of cells or tissues that are part of the skin of a living subject, such as stem cells which are capable to differentiate into fully mature skin cells. As shown in the Examples hereafter, skin regeneration can be assessed by measuring the extent of re-epithelization and/or granulation tissue formation of a skin biopsy sample, or by any other conventional method in the art (Zhang et al., 2015; Braiman-Wiksman et al., 2007). The above contemplated uses encompass treatment of scarification or repeated scarification. These scares may be self-inflicted or may result from chronic inflammatory or autoimmune disorders. In one embodiment, "self-scarification" is scratching, etching, burning, or superficially cutting designs, pictures, or words into the skin, tattooing, as a permanent body modification. In another embodiment, the invention is directed to the use or a method of treatment of self inflicted skin lesions (SISL) in patients afflicted with particular psychiatric disorders, or with ASD, comprising the administration of a suitable amount of a 5-HT1BR activating agent as defined herein. It concerns the administration more specifically in patients with compulsive and impulsive skin picking; impulse control disorders; obsessive compulsive spectrum disorders; trichotillomania; dermatitis art facta; factitial dermatitis; acne excoriée; and neurotic and psychogenic excoriations. In a specific embodiment, the treatment is systemic or local and is applied to patients not previously treated with a 5-HT1BR activating agent, such as fluoxetine or vortioxetine for example.

As fully described hereafter, the present invention provides a solution to accelerate tissue and skin repairs and improve the function as well as improve tissue and skin structure to a significant extent leading to restoring significantly the original and natural appearance, structure, function of the tissue and skin, or the three of them.

Applied to the above defined SISL patients, the compounds of the invention can be administered as a local treatment of the skin in the form a a patch, cream, ointment, band-aid or sticking plaster alone or in combination with a systemic administration of said compounds. It therefore relates to a kit comprising a first product for local administration of the agent defined herein and a second product for systemic administration of the agent defined herein, wherein both first and second products are packaged for simultaneous, separate or concomitant use by the patients.

In a general embodiment, the invention provides a 5-HT1BR activating agent, such as fluoxetine or vortioxetine to delay or slow down skin or hair ageing in promoting stem cells to accelerate tissue, hair or skin natural reconstruction or replacement. More specifically the invention moves the balance between natural or induced senescence in favor of tissue or skin replacement, this without observing exhaustion of the reservoir of stem cells. In this aspect, the invention relates to a method for acceleration of tissue, hair or skin repairs, to improve the function as well as improve tissue, hair, or skin structure to an extent leading to restoring significantly the original and natural appearance, structure, function or the three of them.

By "restoring significantly the original and natural appearance, structure, function (or the three of them) of tissue, hair or skin" it is meant herein and for example increasing by at least 4%, 5% or 10% the number of hair after a period of time, slowing by at least 10% or 20% the rate of hair loss, decreasing the depth of riddles by at least 10%, 20% or even 30%, restoring tissue function by at least 10%, 20% or even 30%.

By "hair repair" or "hair regeneration", it is meant herein repair or regeneration of cells or tissues that are part of the hair of a living subject. Hair repair or regeneration can occur more particularly by hair growth.

The term "hair growth" is defined herein as the maintenance, induction, or stimulation of hair production or genesis in a living subject, on any part of the body. This term can encompass the growth of deficient hair, the extension of the anagen stage of the hair cycle, and/or the conversion of vellus hair to terminal hair. An increase in hair size and/or hair follicle length is however not always associated with hair growth. As shown in the Examples hereafter, hair growth can be assessed by assessing skin color, or by any other conventional method in the art (Müller-Röver et al., 2001).

According to a preferred embodiment, said skin and/or hair repair or regeneration is induced by an external skin and/or hair loss, damage or impairment in a subject, or, in other words, is associated with a skin and/or hair loss, damage or impairment that has been deliberately induced in a subject.

Thus, in the context of the present invention, said skin and/or hair loss, damage or impairment can be either inherent to the subject, or voluntarily induced in said subject. For example, the subject can be either (inherently) suffering from a pathological condition and wishes to repair the skin and/or hair loss, damage or impairment resulting from said condition, or the subject is not suffering from a pathological skin and/or hair loss, damage or impairment and wishes to (voluntarily) induce skin and/or hair loss, damage or impairment so as to promote natural skin and/or hair repair.

In one particular aspect, the invention is directed to the agent herein defined for use in a method of treatment or prevention thereof for slowing down hair loss. An increase of the number of hair above +4% as compared with untreated subjects is considered in the art as a significant slow down of hair loss. This encompasses natural and induced hair deeming or loss over time. It also relates to a method or use of said agents for accelerating stem cells activation in tissue, hair or skin. Such mobilization with no exhaustion of the reservoir of stem cells allows the uses, methods, and/or compositions according to the invention to be applied for weeks, months or even years.

For hair, the method and use above can be combined, in the same product or in separate products as a package, with any and all active ingredients or supplements known in the art to have a benefic action on the hair strength or renewal. These ingredients can be for example stemoxydin, aminexil, minoxidin or Finasterin as well as zinc, magnesium, vitamins, or amino acids such as arginine, or natural extracts. The invention also encompasses the systemic administration of the agent as defined herein in patients that are naïve to 5HT1BR activating agents. As detailed below, said systemic agent is preferably formulated or modified so as not to cross the blood brain barrier (BBB). The systemic administration product can be packaged with a local treatment product as defined herein for all patients.

For skin, the method and use above can be combined, in the same product or in separate products as a package, with any and all active ingredients or supplements known in the art to have a benefic action on the skin strength, tensile, hydration or renewal. These ingredients can be for example hyaluronic acid, vitamins, amino acids such as arginine, or natural extracts. The invention also encompasses the systemic administration of the agent as defined herein in patients that are naïve to a 5-HT1BR activating agents. As detailed below, said systemic agent is preferably formulated or modified so as not to cross the blood brain barrier (BBB). The systemic administration product can be packaged with a local treatment product as defined herein for all patients.

The above products, or package of products can be used also in tissues requiring deep repair, such as for example severe burns or wounds, or repeated wounding such as bedscar, especially before it is going into necrosis in elderly persons or persons immobilized in bed. The product for local administration is applied to significant areas requiring skin graft or in patients undergoing scalp graft as a pretreatment, post-treatment or both.

In another particular aspect, the invention encompasses a systemic administration of the agent as defined herein or a local administration at the areas receiving the engraftment of skin, tissue or scalp prior, after (or both) to the engraftment. Graft material such as skin or scalp can also be pretreated with said agent before surgery in one embodiment.

In the present application, the terms "package", "package product" and "packaged product" are used interchangeably.

In another particular aspect, the invention encompasses a kit or package wherein the compounds of the invention can be administered as a local treatment of the skin in the form of a patch, cream, ointment, band-aid or sticking plaster, alone or in combination with a systemic administration of said compounds. It therefore relates to a kit or package comprising a first product for local administration of the agent defined herein and a second product for systemic administration of the agent defined herein, wherein both first and second products are packaged for simultaneous, separate or concomitant use ; and wherein the first product is formulated for local administration at the areas receiving the engraftment of skin, tissue or scalp prior, after (or both) to the engraftment and the second product is for a systemic administration such as oral route or subcutaneous (sc) injection in the areas to be treated. Graft material such as skin or scalp can also be pretreated with said agent before surgery in one embodiment and can be added in the package as a third product. The invention also relates to a kit or package product comprising the first and third product or second and third product as mentioned above. The invention also relates to the third product as mentioned above, comprising an agent stimulating the 5-HT1BR, such as fluoxetine or vortioxetine, formulated for subcutaneous administration and pre-containing a seringe or an empty seringe. Said third product may contain a seringe filled with said stimulating agent (hereafter called "pre-containing seringe") or an empty seringe. This product can be used for *ex vivo* pretreating a graft before engraftement to the patient, or for *in vivo* preparing a graft in a donor subject or the area to be grafted on the patient, before surgery occurs.

The invention relates to a "pre-containing seringe" comprising an agent stimulating the 5-HT1BR as defined herein in an effective amount to accelerate tissue, hair or skin repairs, to improve the tissue, hair, or skin function or improve tissue, hair, or skin structure to an extent leading to restoring significantly the original and natural appearance, structure, or function of the tissue, hair, or skin, or the three of them (appearance, function and structure). In one aspect, the fluid contained in the seringe is formulated for subcutaneous administration. This package comprises in one embodiment the stimulating agent fluoxetine or vortioxetine. The invention also embraces a pre-containing seringe comprising the agent defined herein in an effective amount to accelerate tissue, hair or skin repairs, to improve the tissue, hair, or skin function or improve tissue, hair, or skin structure to an extent leading to restoring significantly the original and natural appearance, structure, or function of the tissue, hair, or skin, or the three of them (appearance, function and structure). Preferably, the agent in the seringe is fluoxetine or vortioxetine; and the seringe can be provided in a package comprising at least 6, 12 or 24 pre-contained seringes as defined herein above.

By "restoring significantly the original and natural appearance, structure, or function (or the three of them) of tissue, hair or skin", it is meant herein and for example increasing by at least 4%, 5% or 10% the number of hair after a period of time, slowing by at least 10% or 20% the rate of hair loss, decreasing the depth of riddles by at least 10%, 20% or even 30%, restoring tissue function by at least 10%, 20% or even 30%.

According to a preferred embodiment, said skin and/or hair loss, damage or impairment results from a pathological condition in said subject. In this case, said skin and/or hair loss, damage or impairment is said to be "pathological".

In this preferred embodiment, the present invention is directed to a 5-hydroxytryptamine 1B receptor (5-HT1 BR)-stimulating agent for use for treating pathological skin and/or hair loss, damage or impairment.

By "treating", it is herein meant preventing, ameliorating, inhibiting, or curing a deficiency, dysfunction, disease, or other deleterious process, including those that interfere and/or result from therapy.

The term "pathological skin and/or hair loss, damage or impairment" encompasses any type of skin and/or hair loss, damage or impairment that does not result from a natural skin and/or hair loss, damage or impairment. This skin and/or hair loss, damage or impairment affects the health and/or integrity of the subject suffering from said loss, damage or impairment. For example, pathological loss, damage or impairment of skin and/or hair can result either from a disease or disorder, and/or from a trauma or injury to the skin and/or hair. In the latter case, said trauma or injury is preferably substantial. Besides, said pathological skin and/or hair loss, damage or impairment is also referred herein as a "passive skin and/or hair loss, damage or impairment", since it is inherent to the subject (i.e. not voluntarily induced).

Examples of pathological skin loss, damage or impairment include, without limitation, skin redness or soreness, irritated skin, blisters, wounds (such as open wounds, closed wounds, acute wounds, or chronic wounds), burns, abscess, bedsores and/or skin ulcer.

Wounds are a particularly preferred pathological skin loss, damage or impairment to be repaired according to the invention. Accordingly, the invention preferably relates to the use of a 5-hydroxytryptamine 1B receptor (5-HT1 BR)-stimulating agent for boosting the repair, repairing or healing wounds in a subject in need thereof. More precisely, the invention relates to the use of a 5-hydroxytryptamine 1B receptor (5-HT1 BR)-stimulating agent as described herein, for manufacturing a medicament intended to boost the repair, repair or heal wounds, in a subject in need thereof. In other words, the invention relates to a method for boosting the repair, repairing and/or or healing wounds, in a subject in need thereof, comprising the step of administering an effective amount of a 5-hydroxytryptamine 1B receptor (5-HT1 BR)-stimulating agent as described herein, to said subject.

Examples of pathological hair loss, damage or impairment include, without limitation, alopecia and related disorders. Alopecia refers more particularly to the pathological partial or total loss of hair in skin areas in which it is normally present. It typically occurs when the pilar cycle is disturbed, for example as a result of genetic factors, local or systemic disease, febrile conditions, hormonal problems, and secondary effects of drugs. The most frequent phenomenon is a shortening of the hair growth cycle or anagen phase due to cessation of cell proliferation. This results in early onset of the catagen phase, and consequently a large number of hairs in the telogen phase during which the hair follicles are detached from the dermal papillae, and the hairs fall out. Alopecia has a large number of etiologies, including genetic factors, aging, local and systemic diseases, febrile conditions, mental stresses, hormonal problems, and secondary effects of drugs. Different types of alopecia have been identified, which include, without limitation, androgenic alopecia, alopecia totalis, alopecia universalis, alopecia barbae, alopecia mucinosa, post-menopause alopecia, post-pregnancy alopecia, disease-induced alopecia (resulting for example from an infection, skin disease, a tumor, endocrine disorder, metabolic disorder, nutritional disorder), drug-induced alopecia (also known as iatrogenic alopecia, resulting for example from chemotherapy), toxic alopecia, stress-induced alopecia, alopecia cicatrisata or scarring alopecia, alopecia aerata or pseudopelade of Brocq, congenital alopecia, anagen effluvium, telogen effluvium, traction alopecia, and trichotillomania.

Alopecia is a particularly preferred pathological hair loss, damage or impairment to be repaired or treated according to the invention. Accordingly, the invention preferably relates to the use of a 5-hydroxytryptamine 1B receptor (5-HT1 BR)-stimulating agent for treating or repairing alopecia in a subject in need thereof. It also relates to a 5-hydroxytryptamine 1B receptor (5-HT1 BR)-stimulating agent for use for treating or repairing alopecia in a subject in need thereof. In other words, the invention relates to a method for treating or repairing alopecia, in a subject in need thereof, comprising the step of administering an effective amount of a 5-hydroxytryptamine 1B receptor (5-HT1 BR)-stimulating agent as described herein, to said subject.

A more particularly preferred alopecia according to the invention is alopecia involving inflammation of the scalp, such as scarring alopecia.

According to another preferred embodiment, said skin and/or hair loss, damage or impairment results from a deliberate action applied on the subject. In this case, said skin and/or hair loss, damage or impairment is said to be "induced".

Preferably, said skin and/or hair loss, damage or impairment is induced (e.g., mechanically) in a subject. Indeed, the Inventors have discovered that the repair of skin and/or hair can be greatly accelerated when skin and/or hair loss, damage or impairment is performed in combination with the administration of a 5-HT1 BR-stimulating agent. This preferred embodiment is particularly advantageous should one wish to solely attenuate and/or counter natural skin and/or hair loss, damage or impairment, such as skin ageing or natural hair loss.

In this preferred embodiment, the present invention is directed to the 5-hydroxytryptamine 1B receptor (5-HT1 BR)-stimulating agent for use in repairing induced skin and/or hair loss, damage or impairment.

The term "induced skin and/or hair loss, damage or impairment" encompasses herein any type of skin and/or hair loss, damage or impairment that does not result from a natural process, and which does not affect, or not substantially affect, the health and/or integrity of the subject. That is, the subject does not suffer from a pathological skin loss, damage or impairment. Preferably, the subject is healthy.

In another embodiment of the invention, said skin and/or hair loss, damage or impairment is induced (e.g., mechanically) in a subject suffering from a pathological skin loss, damage or impairment or self inflicted skin lesions (SISL). In this case, the invention relates to a 5-hydroxytryptamine 1B receptor (5-HT1 BR)-stimulating agent for use for repairing induced skin and/or hair loss, damage or impairment in a subject suffering from a pathological skin loss, damage or impairment as defined above, or SISL.

Besides, said induced skin and/or hair loss, damage or impairment is also referred herein as a "active skin and/or hair loss, damage or impairment", since it is voluntarily induced to the subject (i.e. not inherent to said subject).

"Induced skin and/or hair loss, damage or impairment" can be obtained by using mechanical and/or physical means such as abrasion, incision, injection, perforation, peeling, hair depilation or shaving, electrical stimulation, laser, hair or skin graft, or surgery.

Examples of means inducing skin and/or hair loss, damage or impairment suitable for this purpose of the invention include, without limitation, abrasion such as micro-abrasion or microdermabrasion, incision such as micro-incision, injection such as microinjection, perforation such as microperforation, peeling, hair depilation or shaving, electrical stimulation, laser, hair or skin graft, surgery, and combinations thereof.

By "effective amount", it is more particularly meant that the agent of the invention is administered in a quantity sufficient to provide the effect for which it is indicated, i.e. skin and/or hair repair, which can be assessed as described above. For example, said 5-HT1 BR-stimulating agent can be administered to the subject at an effective amount comprised between about 5 mg/kg and about 30 mg/kg, preferably between about 10 mg/kg and about 25 mg/kg, more preferably between about 15 mg/kg and about 20 mg/kg and most preferably at about 18 mg/kg, in particular if said agent is administered orally. Yet, according to another preferred embodiment, said 5-HT1 BR-stimulating agent can be administered to the subject at an effective amount comprised between about 0.5 µM and about 100 µM, preferably between 1 µM and about 50 µM, more preferably between about 5 µM and about 25 µM and most preferably at about 10 µM, in particular if said agent is administered topically. It is within the skill of the person in the art to determine the desired amount of 5-HT1 BR-stimulating agent to deliver by routine methods in the art.

The 5-hydroxytryptamine 1B receptor (5-HT1 BR)-stimulating agent according to the invention can also be employed in a therapeutic or cosmetic composition which may comprise additional active agent(s).

Accordingly, in another aspect, the invention further provides a therapeutic composition for use as a booster of skin and/or hair repair, wherein said composition comprises at least one 5-hydroxytryptamine 1B receptor (5-HT1 BR)-stimulating agent and at least one acceptable excipient. Specifically, said composition is for use for boosting the skin and/or hair repair; or repairing skin and/or hair loss, damage or impairment; or treating pathological skin and/or hair loss, damage or impairment (such as alopecia).

In another aspect, the invention further provides the composition comprising at least one 5-hydroxytryptamine 1B receptor (5-HT1 BR)-stimulating agent and at least one acceptable excipient, for use in boosting the skin and/or hair repair or repairing the skin and/or hair, or regenerating the skin and/or hair in a subject.

It also provides the composition comprising at least one 5-hydroxytryptamine 1B receptor (5-HT1 BR)-stimulating agent and at least one acceptable excipient for use in regenerating skin and/or hair or repairing or boosting the repair of induced skin and/or hair loss, damage or impairment.

In other words, the invention relates to the compounds for use in a method for regenerating skin and/or hair or repairing or boosting the repair of skin and/or hair loss, damage or impairment, comprising the step of administering an effective amount of said composition to said subject.

The "acceptable excipient" that is comprised in said compositions is an inactive or inert, and therefore nontoxic, component, as it has no physiological action, and can be used to improve properties of said composition, such as shelf-life, retention time at the application site, consumer acceptance, etc. Such excipients include, without limitation, surfactants (cationic, anionic, or neutral); surface stabilizers; other enhancers, such as preservatives, wetting or emulsifying agents; solvents; buffers; salt solutions; dispersion medium; isotonic and absorption delaying agents, and the like; that are physiologically compatible.

According to a particularly preferred embodiment, said compositions further comprise at least one active agent, which may also favor skin and/or hair repair. Such agents are well-known in the art.

Examples of active agents which are suitable with a 5-hydroxytryptamine 1B receptor (5-HT1 BR)-stimulating agent for the purpose of the invention, whether for skin and/or hair repair, include antioxidants such as ascorbyl palmitate liposomal, sodium ascorbyl phosphate (INCI), ubiquinol (i.e. coenzyme Q10), lipoic acid such as alpha-lipoic acid (ALA), uric acid such as ferulic acid, carotenes (e.g. beta-carotene), lutein, lycopene, selenium, coffee acid, quercetin, rutin, buthylhydroxyanizole (BHA), buthylhydroxytoluene (BHT), fruit acids (AHA acids) such as glycolic acid and lactic acid; vitamins, precursors and derivatives thereof such as vitamin E (a-tocopherol, which is also an antioxidant) and its derivative tocopheryl acetate, vitamin A (ascorbic acid, which is also an antioxidant), retinoids such as retinoic acid and retinol, vitamin B2 (ribloflavin), vitamin B3 (niacide and niacinamide), provitamine B5 (panthotenol), vitamin B5 (panthotenic acid), D-calcium pantothenate, vitamin B6 (pyridoxine), pyridoxine hydrochloride, vitamin B8 (biotin), vitamin B9 (folic acid), vitamin B10 (para-aminobenzoic acid), vitamin B12 (cobalamin), vitamin C (also an antioxidant), inositol, myo-inositol; phytohormones such as phytoestrogens, auxin, ethylene, jasmonic acid (JA), brassinosteroid (BR), and strigolactone (SL); peptides such as glutathione (also an antioxidant), collagen, elastin, wheat extract; essential and non-essential amino-acids; essential fatty acids such as linoleic acid and alphalinoleic acid; azelaic acid; 5-lipoxygenase inhibitors; tyrosinase inhibitors; hair matrix cell activators such as the compounds described in patent application US20020052498 (incorporated by reference) and matrix metalloproteinase inhibitors such as the ones described in patent application US20040071647 (incorporated by reference); trace elements such as copper, zinc, selenium (also an antioxidant), manganese and silicon; and combinations thereof.

Active agents which are known to more particularly promote skin repair, include, without limitation, cortisteroids, plant extracts from *Aloe Vera*, *Hippophaerhamnoides L., Catharanthus roseus plant, Lycopodium serratum, Sesamum indicum, Radixpaeoniae, Morinda citrifolia Linn, Termina liabelliricaRoxb, Moringaoleifera Linn,* naltrexone, plasmin, plasmin mixed with deoxyribonuclease, fibrin, silver nitrate, growth factors, ecabet sodium, placenta extract, thrombin, becaplermin, and combinations thereof.

Active agents which are known to more particularly promote hair repair include, without limitation, vasodilators such as minoxidil and compounds described in patent applications US20040157856 and US20050053572; anti-androgens such as finasteride and compounds described in patent applications US20060009430, US20060009427, US20050085467, US20050118282, US20060009429, US20030007941, US20030073616 and EP0279010; agents acting as both vasodilatators and anti-androgens such as the compounds described in patent applications US20060052405, US20050123577, US6447762; psoralen when combined with PUVA therapy; contact allergens such as dinitrochlorobenzene (DNCB), squaric acid dibutylester (SADBE) and diphencyprone (DPCP), and combinations thereof.

It is within the skill of ordinary person in the art to select the appropriate combination of 5-HT1 BR-stimulating agent and active agent, for the purpose of the invention.

As indicated above, it is particularly advantageous to combine a 5-hydroxytryptamine 1B receptor (5-HT1 BR)-stimulating agent according to the invention with an active agent as described above.

It is thus another aspect of the invention to provide a 5-hydroxytryptamine 1B receptor (5-HT1 BR)-stimulating agent and an active agent, as a combined therapeutic preparation for simultaneous, separate or sequential administration in a subject in need of skin and/or hair repair.

Specifically, this combined product is for use for repairing or treating pathological skin and/or hair loss, damage or impairment (such as alopecia).

The active agent is as described above.

In another aspect, the invention relates to the claimed compounds and compositions for use in a therapeutic method for treating a subject, comprising:
a) administering to said subject at least one 5-hydroxytryptamine 1B receptor (5-HT1 BR)-stimulating agent; and
b) further administering to said subject at least one active agent, simultaneously, separately or sequentially to (i.e. before or after) step a).

The active agent is as described above.

In this particular aspect, the subject can be in need of skin and/or hair repair. Alternatively, the subject can be healthy or at least does not suffer from a pathological skin loss, damage or impairment. Preferably, the subject does not suffer from any degenerative diseases, nor from any vascular disorders. In particular, the subject does not suffer from diabete mellitus, venous insufficiency or obesity.

In a particular embodiment, the invention relates to the claimed compounds and compositions for use in a method for boosting skin and/or hair repair or regenerating skin and/or hair in a subject, said method comprising:
a) administering to said subject at least one 5-hydroxytryptamine 1B receptor (5-HT1 BR)-stimulating agent; and
b) further administering to said subject at least one active agent, simultaneously, separately or sequentially to (i.e. before or after) step a).

The active agent is as described above.

In this particular embodiment, the subject does not suffer from a pathological skin loss, damage or impairment. Preferably, the subject does not suffer from any degenerative diseases, nor from any vascular disorders. In particular, the subject does not suffer from diabete mellitus, venous insufficiency or obesity. More preferably, the subject is healthy.

In another particular embodiment, the invention relates to the claimed compounds and compositions for use in a therapeutic method for repairing or treating pathological skin loss, damage or impairment in a subject, said method comprising:
a) administering to said subject at least one 5-hydroxytryptamine 1B receptor (5-HT1 BR)-stimulating agent; and
b) further administering to said subject at least one active agent, simultaneously, separately or sequentially to (i.e. before or after) step a).

The active agent is as described above.

In this particular embodiment, the subject suffers from a pathological skin loss, damage or impairment as defined above. Yet, preferably, the subject does not suffer from any degenerative diseases, nor from any vascular disorders. In particular, the subject does not suffer from diabete mellitus, venous insufficiency or obesity.

As illustrated in the Examples hereafter, it can also be advantageous to combine a 5-hydroxytryptamine 1B receptor (5-HT1 BR)-stimulating agent according to the invention with an external means for inducing skin and/or hair loss, damage or impairment.

Said external means is for example a mechanical and/or physical means such as abrasion, incision, injection, perforation, peeling, hair depilation or shaving, electrical stimulation, laser, hair or skin graft, or surgery.

It is thus another aspect of the invention to provide a 5-hydroxytryptamine 1B receptor (5-HT1 BR)-stimulating agent and an external means for inducing skin and/or hair loss, damage or impairment, for simultaneous, separate or sequential administration in a subject. In other words, the invention relates to the claimed compounds and compositions for use in a method for treating a subject in need thereof, comprising:
a) administering to said subject at least one 5-hydroxytryptamine 1B receptor (5-HT1 BR)-stimulating agent; and
b) further administering to said subject at least one means for inducing skin and/or hair loss, damage or impairment, simultaneously, separately or sequentially to (i.e. before or after) step a), said means for inducing skin and/or hair loss, damage or impairment being chosen from: abrasion, injection, peeling, depilation, shaving, or laser.

In this particular aspect, the subject can be in need of skin and/or hair repair as it is suffering from a pathological skin and/or hair loss, damage or impairment as defined above. Preferably, the subject does not suffer from any degenerative diseases, nor from any vascular disorders. In particular, the subject does not suffer from diabete mellitus, venous insufficiency or obesity.

In another aspect, the present disclosure is directed to the cosmetic use of a 5-hydroxytryptamine 1B receptor (5-HT1 BR)-stimulating agent as a booster of skin and/or hair repair, or for repairing or regenerating skin and/or hair:
- in conjunction with passive skin and/or hair loss, damage or impairment, such as skin redness or soreness, irritated skin, blisters, wounds, burns, abscess, bedsores and/or skin ulcer or alopecia, or
- in conjunction with an active skin and/or hair loss, damage or impairment by a means for inducing skin and/or hair loss, damage or impairment, said means being as described above.

In this latter case, the invention relates to the claimed compounds or compositions for use in a method for regenerating or repairing skin and/or hair or boosting the skin and/or hair repair in a subject, comprising:
a) administering to said subject at least one 5-hydroxytryptamine 1B receptor (5-HT1 BR)-stimulating agent; and
b) further administering to said subject at least one means for inducing skin and/or hair loss, damage or impairment, simultaneously, separately or sequentially to (i.e. before or after) step a), said means being chosen from: abrasion, injection, peeling, depilation, shaving, or laser.

In this embodiment, the subject can be healthy or at least does not suffer from a pathological skin loss, damage or impairment. Preferably, the subject does not suffer from any degenerative diseases, nor from any vascular disorders. In particular, the subject does not suffer from diabete mellitus, venous insufficiency or obesity.

Methods for administering to individuals the 5-hydroxytryptamine 1B receptor (5-HT1 BR)-stimulating agent or composition according to the invention are well known to those skilled in the art. Such methods include, but are not limited to, parenteral administration (e.g., intra-dermal, intravenous, subcutaneous, etc.), or topical application (e.g., on skin areas such as wounds, burns, etc., or on skin displaying hair loss, damage or impairment).

A local administration to the site of interest is herein particularly preferred, said site being herein preferably the skin whether one wish to repair skin and/or hair (i.e. topical administration), though an oral administration is also suitable for the purpose of the invention. A topical administration is even more particularly preferred.

Accordingly, the 5-hydroxytryptamine 1B receptor (5-HT1 BR)-stimulating agent or the composition is preferably in a form suitable for the purpose of the invention. For example, said agent or composition may be in a form suitable for parenteral, oral (i.e. enteral or *per* os) or local administration (e.g. topical administration), such as a liquid suspension, a solid dosage form (granules, pills, capsules or tablets), or a paste or gel.

For topical application, the 5-hydroxytryptamine 1B receptor (5-HT1 BR)-stimulating agent or composition of the invention can have the form of aqueous or oily solutions or of lotion- or serum-type dispersions, milk-type emulsions with a liquid or semi-liquid consistency, obtained by dispersion of an aqueous phase in a silicone phase (W/Si), of a fatty phase in an aqueous phase (O/W: oil-in-water emulsion) or, conversely, of an aqueous phase in a fatty phase (W/O: water-in-oil emulsion), or aqueous or anhydrous suspensions or emulsions with a cream- or gel-type soft consistency, or of microcapsules or microparticles, or of ionic and/or non-ionic-type vesicular dispersions, or of foams. These agents or compositions can be prepared using conventional methods in the art.

The 5-HT1 BR-stimulating agent or composition of the invention can not only be in the form of shampoo for cleansing, protecting, treating or caring for the hair, or of cream for cleansing, protecting, treating or caring for the skin (for example, day creams, night creams, makeup remover creams, foundation creams, sunscreen creams), but also as liquid foundations, makeup remover milks, body protection or care milks, sunscreen milks, skincare lotions, gels or foams, such as cleansing lotions, sunscreen lotions, artificial tanning lotions, compositions for the bath, deodorant compositions comprising e.g. a bactericide, aftershave gels or lotions, depilatory creams.

The 5-HT1 BR-stimulating agent or composition according to the invention can also consist of powder or non-powder solid preparations, for example in the form of a stick, a compacted powder, cleansing soaps or bars. It can be provided also as patches, brushes and applicators allowing localized application on skin or hair. It can be used as a care product or as a makeup product.

The scheme of administration of the 5-hydroxytryptamine 1B receptor (5-HT1 BR)-stimulating agent or of the composition according to the invention can be adapted by the skilled person in the art depending on the age, weight and severity of the skin and/or hair loss, damage or impairment to be repaired.

Accordingly, the 5-hydroxytryptamine 1B receptor (5-HT1 BR) or the composition according to the invention can be administered once a day, preferably for a period of about 6 weeks (in particular for slow-acting 5-HT1 BR-stimulating agents such as fluoxetine) or for a period of about 12 days (in particular for fast-acting 5-HT1 BR-stimulating agents such as vortioxetine).

### Therapeutic applications according to the invention

It is more particularly proposed herein to use agents stimulating 5-HT1 BR activity, as novel therapeutic drugs for stimulating skin and/or hair repair in patients in need thereof, or, in other words, in patients suffering from, or at risk of suffering from skin and/or hair loss, damage or impairment.

Thus, in this aspect, the present invention is directed to a 5-hydroxytryptamine 1B receptor (5-HT1 BR)-stimulating agent, for therapeutic use in the boosting and/or promotion and/or acceleration of skin and/or hair repair, or for use in the repair and/or treatment of pathological skin and/or hair loss, damage or impairment, in a subject in need thereof.

In other words, the invention relates to the claimed compounds or compositions for use in a therapeutic method for boosting and/or promoting and/or accelerating skin and/or hair repair, or to a method for repairing and/or treating pathological skin and/or hair loss, damage or impairment, in a subject in need thereof, comprising the step of administering an effective amount of a 5-hydroxytryptamine 1B receptor (5-HT1 BR)-stimulating agent as described herein, to said subject.

The term "treating, "treatment" or "treat" as used herein encompasses, among other, preventing, ameliorating, inhibiting, or curing a deficiency, dysfunction, disease, or other deleterious process, including those that interfere and/or result from therapy.

With regard to the therapeutic aspect of the invention, it should be understood that "a subject in need thereof" refers more particularly to a subject, as defined above, in need of a therapy which boosts and/or promotes and/or accelerates skin and/or hair repair, or which needs treatment for pathological skin and/or hair loss, damage or impairment; in other words, said subject suffers from a disease which can be treated by promoting and/or accelerating skin and/or hair repair, or from a pathological skin and/or hair loss, damage or impairment. Preferably, said subject does not suffer from any degenerative diseases, nor from any vascular disorders. In particular, the subject does not suffer from diabete mellitus, venous insufficiency or obesity.

As mentioned above, "a subject in need thereof" in the context of the pathological uses of the present compositions can be patients afflicted with particular psychiatric disorders, such as Autism Spectrum Disorders (ASD).

These patients may indeed suffer from compulsive and impulsive skin picking; impulse control disorders; obsessive compulsive spectrum disorders; trichotillomania; dermatitis art facta; factitial dermatitis; acne excoriée; and neurotic and psychogenic excoriations.

These patients may suffer from self inflicted skin lesions (SISL). In this case, the invention relates to a 5-hydroxytryptamine 1B receptor (5-HT1 BR)-stimulating agent for use for repairing induced skin and/or hair loss, damage or impairment in a subject suffering from SISL.

In a specific embodiment, the treatment of the invention is systemic or local and is applied to patients that have not been previously treated with a 5-HT1BR activating agent, such as fluoxetine or vortioxetine for example.

Examples of pathological skin loss, damage or impairment include herein, without limitation, skin redness or soreness, irritated skin, blisters, wounds (such as open wounds, closed wounds, acute wounds, or chronic wounds), burns, abscess, bedsores and/or skin ulcer.

Wounds are a particularly preferred pathological skin loss, damage or impairment to be repaired and/or treated according to the invention. Accordingly, the invention preferably relates to the 5-hydroxytryptamine 1B receptor (5-HT1 BR)-stimulating agent for use in boosting the repair of wounds, for healing, repairing and/or treating wounds in a subject in need thereof. In other words, the invention relates to the claimed compounds or compositions for use in therapeutic method for boosting the repair of wounds, for healing, repairing and/or treating wounds, in a subject in need thereof, comprising the step of administering an effective amount of a 5-hydroxytryptamine 1B receptor (5-HT1 BR)-stimulating agent as described herein, to said subject.

Examples of pathological hair loss, damage or impairment include herein, without limitation, alopecia and related disorders, as described above.

Alopecia is a particularly preferred pathological hair loss, damage or impairment to be treated according to the invention. Accordingly, the invention preferably relates to the 5-hydroxytryptamine 1B receptor (5-HT1 BR)-stimulating agent for use in treating alopecia in a subject in need thereof. other words, the invention relates to the claimed compounds or compositions for use in a therapeutic method for treating alopecia, in a subject in need thereof, comprising the step of administering an effective amount of a 5-hydroxytryptamine 1B receptor (5-HT1 BR)-stimulating agent as described herein, to said subject.

A more particularly preferred alopecia according to the invention is alopecia involving inflammation of the scalp, such as scarring alopecia.

According to a preferred embodiment, said 5-HT1 BR-stimulating agent can be administered to the subject at an effective amount comprised between about 5 mg/kg and about 30 mg/kg, preferably between about 10 mg/kg and about 25 mg/kg, more preferably between about 15 mg/kg and about 20 mg/kg and most preferably at about 18 mg/kg, in particular if said agent is administered orally. Yet, according to another preferred embodiment, said 5-HT1 BR-stimulating agent can be administered to the subject at an effective amount comprised between about 0.5 µM and about 100 µM, preferably between 1 µM and about 50 µM, more preferably between about 5 µM and about 25 µM and most preferably at about 10 µM, in particular if said agent is administered topically. It is within the skill of the person in the art to determine the desired therapeutic amount of 5-HT1 BR-stimulating agent to deliver by routine methods in the art.

The 5-hydroxytryptamine 1B receptor (5-HT1 BR)-stimulating agent according to the invention can also be employed for therapeutic use in a pharmaceutical composition which may comprise additional active agent(s).

Accordingly, in another aspect, the invention further provides a pharmaceutical composition for therapeutic use in the boosting and/or promotion and/or acceleration of skin and/or hair repair, or for use in the repair and/or treatment of pathological skin and/or hair loss, damage or impairment, in a subject in need thereof, wherein said composition comprises at least one 5-hydroxytryptamine 1B receptor (5-HT1 BR)-stimulating agent and at least one pharmaceutically acceptable excipient.

In other words, the invention relates to the claimed compounds or compositions use in a therapeutic method for boosting and/or promoting and/or accelerating skin and/or hair repair, or a method for repairing and/or treating pathological skin and/or hair loss, damage or impairment, in a subject in need thereof, comprising the step of administering an effective amount of said composition to said subject.

According to a particularly preferred embodiment, said pharmaceutical composition further comprises at least one active agent, which may also favor skin and/or hair repair, or contribute to treating pathological skin and/or hair loss, damage or impairment. Preferred active agents are as described above.

It is within the skill of ordinary person in the art to select the appropriate combination of 5-HT1 BR-stimulating agent and active agent, for the therapeutic purpose of the invention.

As indicated above, it is particularly advantageous to combine a 5-hydroxytryptamine 1B receptor (5-HT1 BR)-stimulating agent according to the invention with an active agent as described above.

It is thus another aspect of the invention to provide a 5-hydroxytryptamine 1B receptor (5-HT1 BR)-stimulating agent and an active agent, as a combined preparation for simultaneous, separate or sequential administration in a subject in need of a therapy for boosting and/or promoting and/or accelerating skin and/or hair repair, or suffering from a pathological skin and/or hair loss, damage or impairment.

In other words, the invention relates to the claimed compounds or compositions for use in a therapeutic method for treating a subject in need of a therapy promoting and/or accelerating skin and/or hair repair, or suffering from a pathological skin and/or hair loss, damage or impairment, comprising:
a) administering to said subject at least one 5-hydroxytryptamine 1B receptor (5-HT1 BR)-stimulating agent; and
b) further administering to said subject at least one active agent, simultaneously, separately or sequentially to (i.e. before or after) step a).

It is thus another aspect of the invention to provide a 5-hydroxytryptamine 1B receptor (5-HT1 BR)-stimulating agent and an external means for inducing skin and/or hair loss, damage or impairment, said means being chosen from: abrasion, injection, peeling, depilation, shaving, or laser, for simultaneous, separate or sequential administration in a subject.

In other words, the invention relates to a method for treating a subject in need thereof, comprising:
a) administering to said subject at least one 5-hydroxytryptamine 1B receptor (5-HT1 BR)-stimulating agent; and
b) further administering to said subject at least one means inducing skin and/or hair loss, damage or impairment, simultaneously, separately or sequentially to (i.e. before or after) step a).

Preferred means inducing skin and/or hair loss, damage or impairment are as described above. In this particular aspect, the subject can be in need of skin and/or hair repair as it is suffering from a pathological skin and/or hair loss, damage or impairment as defined above. Preferably, the subject does not suffer from any degenerative diseases, nor from any vascular disorders. In particular, the subject does not suffer from diabete mellitus, venous insufficiency or obesity.

In another embodiment of the invention, said skin and/or hair loss, damage or impairment is induced (e.g., mechanically) in a subject suffering from psychiatric disorders involving self inflicted skin lesions (SISL). In this case, the invention relates to a 5-hydroxytryptamine 1B receptor (5-HT1 BR)-stimulating agent for use for repairing induced skin and/or hair loss, damage or impairment in a subject suffering from one of the psychiatric disorders described above.

Methods for administering to individuals the 5-hydroxytryptamine 1B receptor (5-HT1 BR)-stimulating agent or pharmaceutical composition according to the invention, and schemes of administration, are as described above.

Cosmetic applications not encompassed by the invention

Based on the experimental data displayed herein, the skilled person in the art would readily understand that 5-hydroxytryptamine 1B receptor (5-HT1 BR)-stimulating agents can also be used for cosmetic purpose only, in particular for rejuvenating the skin, and/or for limiting natural loss of hair.

Thus, in another aspect, the present disclosure is directed to the cosmetic use of a 5-hydroxytryptamine 1B receptor (5-HT1 BR)-stimulating agent, for boosting and/or promoting and/or accelerating skin and/or hair repair, or for slowing, attenuating and/or countering natural skin and/or hair loss, damage or impairment, in a subject in need thereof.

More precisely, the disclosure relates to the use of a 5-hydroxytryptamine 1B receptor (5-HT1 BR)-stimulating agent as described herein, for manufacturing a cosmetic composition intended to boost and/or promote and/or accelerate skin and/or hair repair, or to slow, attenuate and/or counter natural skin and/or hair loss, damage or impairment, in a subject in need thereof.

In other words, the disclosure relates to a cosmetic method for boosting and/or promoting and/or accelerating skin and/or hair repair, or for slowing, attenuating and/or countering natural skin and/or hair loss, damage or impairment, in a subject in need thereof, comprising the step of administering an effective amount of a 5-hydroxytryptamine 1B receptor (5-HT1 BR)-stimulating agent as described herein, to said subject.

The term "natural skin and/or hair loss, damage or impairment" encompasses any type of skin and/or hair loss, damage or impairment that results from a natural process, and which therefore does not affect the health and/or integrity of the subject. For example, natural loss, damage or impairment of skin and/or hair can result from ageing.

A non-limiting example of natural skin loss, damage or impairment according to the invention is skin ageing.

The expression "skin ageing" is considered herein in its broadest meaning, and can be associated with at least one condition selected from disintegration of collagen fiber bundle structure, formation of wrinkles, loss of skin elasticity, changes in skin texture, and decrease in the difference between a furrow and a rise of the skin surface. The term "skin ageing" encompasses herein intrinsic ageing and extrinsic ageing.

By "intrinsic skin ageing," also known as "normal" or chronobiological ageing, it is meant herein physiological changes at the molecular, cellular and/or tissular level of a subject skin resulting from a programmed senescence involving endogenous factors. This intrinsic ageing causes in particular slowing of the renewal of skin cells, keratinocytes, which is reflected primarily by the appearance of clinical changes such as a decrease in subcutaneous adipose tissue and the appearance of fine lines or wrinkles, and by histological changes such as an increase in the number and thickness of elastic fibers, a loss of vertical fibers of the membrane of elastic tissue, and the presence of large irregular fibroblasts in the cells of this elastic tissue.

By "extrinsic skin ageing", it is meant herein physiological changes at the molecular, cellular and/or tissular level of a subject skin resulting from exposure to exogenous factors such as chemical and physical stimulations that can degrade the normal functions of the skin, but that nevertheless do not induce a pathological skin ageing. Such stimulations include in particular exposure to the sun, light, UVs, stress and malnutrition. This extrinsic ageing results in clinical changes such as deep wrinkles and the formation of skin having lost its firmness, its suppleness and its elasticity. These transformations are due primarily to histological changes, such as an excessive change in elastic tissue in the upper dermis and quantitative and qualitative degeneration of collagen fibers.

Skin ageing, such as fine lines and wrinkles that may appear on the face, neck, low neckline or hands of the subject, is a particularly preferred natural skin loss, damage or impairment to be slowed, attenuated and/or countered according to the invention. These signs of ageing may be smoothed by the 5-hydroxytryptamine 1B receptor (5-HT1 BR)-stimulating agent of the invention. Accordingly, the invention preferably relates to the cosmetic use of a 5-hydroxytryptamine 1B receptor (5-HT1 BR)-stimulating agent for slowing, attenuating and/or countering skin ageing, in a subject in need thereof. More precisely, the invention relates to the use of a 5-hydroxytryptamine 1B receptor (5-HT1 BR)-stimulating agent as described herein, for manufacturing a cosmetic composition intended to slow, attenuate and/or counter skin ageing in a subject in need thereof. In other words, the invention relates to a cosmetic method for slowing, attenuating and/or countering skin ageing in a subject in need thereof, comprising the step of administering an effective amount of a 5-hydroxytryptamine 1B receptor (5-HT1 BR)-stimulating agent as described herein, to said subject.

The disclosure relates to the cosmetic use of a 5-hydroxytryptamine 1B receptor (5-HT1 BR)-stimulating agent for slowing, attenuating and/or countering natural hair loss, in a subject in need thereof. More precisely, the disclosure relates to the use of a 5-hydroxytryptamine 1B receptor (5-HT1 BR)-stimulating agent as described herein, for manufacturing a cosmetic composition intended to slow, attenuate and/or counter natural hair loss in a subject in need thereof. In other words, the disclosure relates to a cosmetic method for slowing, attenuating and/or countering natural hair loss in a subject in need thereof, comprising the step of administering an effective amount of a 5-hydroxytryptamine 1B receptor (5-HT1 BR)-stimulating agent as described herein, to said subject.

"Natural hair loss" can more particularly be defined herein as the loss of hair observed in a subject exhibiting a physiologically normal pilar cycle (i.e. non disturbed pilar cycle). More preferably, the hair loss in such subject is of about a hundred hair per day and/or the hair density of said subject if of about 200 to about 300 hair/cm² on the scalp, of about 50 hair/cm² on the face, and/or of about 10 hair/cm² on the face. Yet, even more preferably, said subject is not suffering from alopecia.

With regard to the cosmetic aspect of the disclosure it should be understood that "a subject in need thereof" refers more particularly to a subject in need of a cosmetic treatment boosting and/or promoting and/or accelerating skin and/or hair repair, or which desires to slow, attenuate and/or counter natural skin and/or hair loss, damage or impairment; in other words, said subject does not suffer from a pathological skin and/or hair loss, damage or impairment. In this sense, said subject can be considered as "healthy".

In this aspect, the subject preferably does not suffer from any degenerative diseases, nor from any vascular disorders. In particular, the subject does not suffer from diabete mellitus, venous insufficiency or obesity.

The disclosure also relates to a cosmetic method for regenerating or repairing skin and/or hair and/or for promoting and/or accelerating and/or boosting the skin and/or hair repair in a subject, comprising administering to said subject at least one 5-hydroxytryptamine 1B receptor (5-HT1 BR)-stimulating agent.

According to the disclosure, said 5-HT1 BR-stimulating agent can be administered to the subject at an effective amount comprised between about 5 mg/kg and about 30 mg/kg, preferably between about 10 mg/kg and about 25 mg/kg, more preferably between about 15 mg/kg and about 20 mg/kg and most preferably at about 18 mg/kg, in particular if said agent is administered orally. Yet, according to the disclosure, said 5-HT1 BR-stimulating agent can be administered to the subject at an effective amount comprised between about 0.5 µM and about 100 µM, preferably between 1 µM and about 50 µM, more preferably between about 5 µM and about 25 µM and most preferably at about 10 µM, in particular if said agent is administered topically. It is within the skill of the person in the art to determine the desired cosmetic amount of 5-HT1 BR-stimulating agent to deliver by routine methods in the art.

The 5-hydroxytryptamine 1B receptor (5-HT1 BR)-stimulating agent according to the disclosure can also be employed in a cosmetic composition which may comprise additional active agent(s).

Accordingly, the disclosure further relates to the use of a cosmetic composition, for promoting and/or accelerating skin and/or hair repair, or for slowing, attenuating and/or countering natural skin and/or hair loss, damage or impairment, in a subject in need thereof, wherein said composition comprises at least one 5-hydroxytryptamine 1B receptor (5-HT1 BR)-stimulating agent and at least one cosmetically acceptable excipient.

More precisely, the disclosure relates to a cosmetic method for promoting and/or accelerating skin and/or hair repair, or for slowing, attenuating and/or countering natural skin and/or hair loss, damage or impairment, in a subject in need thereof, comprising the step of administering an effective amount of said composition to said subject.

According to the disclosure, said cosmetic composition further comprises at least one active agent, which may also favor skin and/or hair repair, or contribute to slow, attenuate and/or counter natural skin and/or hair loss, damage or impairment. Preferred active agents are as described above.

According to the disclosure, the cosmetic method of the invention can be performed as follows:
a) administering to said subject at least one 5-hydroxytryptamine 1B receptor (5-HT1 BR)-stimulating agent; and
b) further administering to said subject at least one active agent, simultaneously, separately or sequentially to (i.e. before or after) step a).

It is within the skill of ordinary person in the art to select the appropriate combination of 5-HT1 BR-stimulating agent and active agent, for the cosmetic purpose of the disclosure.

As indicated above, it is particularly advantageous to combine a 5-hydroxytryptamine 1B receptor (5-HT1 BR)-stimulating agent according to the disclosure with an active agent.

It is thus another aspect of the disclosure to provide a 5-hydroxytryptamine 1B receptor (5-HT1 BR)-stimulating agent and an active agent, as a combined preparation for simultaneous, separate or sequential administration in a subject in need of a cosmetic treatment promoting and/or accelerating skin and/or hair repair, or slowing, attenuating and/or countering natural skin and/or hair loss, damage or impairment.

In other words, the disclosure relates to a method for cosmetically treating a subject in need of skin and/or hair repair, or of slowing, attenuating and/or countering natural skin and/or hair loss, damage or impairment, comprising:
a) administering to said subject at least one 5-hydroxytryptamine 1B receptor (5-HT1 BR)-stimulating agent; and
b) further administering to said subject at least one active agent, simultaneously, separately or sequentially to (i.e. before or after) step a).

Preferred active agents are as described above.

The disclosure also relates to a cosmetic composition containing at least one 5-hydroxytryptamine 1B receptor (5-HT1 BR)-stimulating agent and an active agent as defined above may be added for use for regenerating or repairing skin and/or hair and/or for promoting and/or accelerating and/or boosting the skin and/or hair repair in a subject. Preferred active agents are as described above.

The disclosure also relates to the use of a cosmetic composition containing at least one 5-hydroxytryptamine 1B receptor (5-HT1 BR)-stimulating agent and an active agent as defined above for regenerating or repairing skin and/or hair and/or for promoting and/or accelerating and/or boosting the skin and/or hair repair in a subject. Preferred active agents are as described above.

In the cosmetic aspect of the disclosure, it is also particularly preferred to combine a 5-hydroxytryptamine 1B receptor (5-HT1 BR)-stimulating agent according to the disclosure with an external means inducing skin and/or hair loss, damage or impairment. Indeed, the Inventors have discovered that the natural repair of skin and/or hair can be greatly accelerated when such induction is performed in combination with the administration of a 5-HT1 BR-stimulating agent.

It is thus another aspect of the disclosure to provide a 5-hydroxytryptamine 1B receptor (5-HT1 BR)-stimulating agent and an external means inducing skin and/or hair loss, damage or impairment, for simultaneous, separate or sequential administration in a subject in need of a cosmetic treatment promoting and/or accelerating skin and/or hair repair, or slowing, attenuating and/or countering natural skin and/or hair loss, damage or impairment.

In other words, the disclosure relates to a method for cosmetically treating a subject in need thereof, comprising:
a) administering to said subject at least one 5-hydroxytryptamine 1B receptor (5-HT1 BR)-stimulating agent; and
b) further administering to said subject at least one means for inducing skin and/or hair loss, damage or impairment, simultaneously, separately or sequentially to (i.e. before or after) step a).

Preferred means inducing skin and/or hair loss, damage or impairment are mechanical and/or physical means such as abrasion, incision, injection, perforation, peeling, hair depilation or shaving, electrical stimulation, laser, hair or skin graft, or surgery, as described above.

The disclosure also relates to a cosmetic method for regenerating or repairing skin and/or hair and/or for promoting and/or accelerating and/or boosting the skin and/or hair repair in a subject, comprising :
a) administering to said subject at least one 5-hydroxytryptamine 1B receptor (5-HT1 BR)-stimulating agent; and
b) further administering to said subject at least one means for inducing skin and/or hair loss, damage or impairment, simultaneously, separately or sequentially to (i.e. before or after) step a).

Said means are as described above.

The disclosure also relates to the use of a cosmetic method for regenerating or repairing skin and/or hair and/or for promoting and/or accelerating and/or boosting the skin and/or hair repair in a subject, comprising :
a) administering to said subject at least one 5-hydroxytryptamine 1B receptor (5-HT1 BR)-stimulating agent; and
b) further administering to said subject at least one means for inducing skin and/or hair loss, damage or impairment, simultaneously, separately or sequentially to (i.e. before or after) step a).

Said means are as described above.

Methods for administering to individuals the 5-hydroxytryptamine 1B receptor (5-HT1 BR)-stimulating agent or cosmetic composition according to the invention, and schemes of administration, are also as described above.

### 5-hydroxytryptamine 1B receptor (5-HT1 BR)-stimulatinq agents

The different aspects and embodiments of the present invention rely on the use of 5-hydroxytryptamine 1B receptor (5-HT1 BR)-stimulating agents, which act directly on 5-HT1 BR.

According to a preferred embodiment, said 5-hydroxytryptamine 1B receptor (5-HT1 BR)-stimulating agent is as described herein, with the proviso that it is not paroxetine.

According to a preferred embodiment, the 5-HT1 BR-stimulating agent used in the present invention is selected from the group consisting of antidepressant agents and antimigraine drugs, pharmaceutically or cosmetically acceptable derivatives, analogs, isomers, metabolites, salts, solvates, clathrates, polymorphs, and co-crystals thereof, and combinations thereof.

By "derivative", it is meant herein a compound that is directly derived from a chemical compound of interest (i.e. 5-HT1 BR-stimulating agent) and is structurally similar though non-identical to said compound, and which retains the same biological activity and/or physico-chemical properties.

By "analog", or "functional analog", it is meant herein a compound that is not directly derived from a chemical compound of interest and is thus structurally different, but exhibits the same biological activity and/or physico-chemical properties, such as isosters.

"Derivatives" and "analogs" of the 5-HT1 BR-stimulating agents according to the invention encompass herein compounds that retain the 5-HT1 BR-stimulating activity as defined above, but that do not cross the blood-brain barrier, as further described below.

By "isomer", it is meant herein a compound having the same chemical formula as a compound of interest, but a different chemical structure. This term encompasses structural isomers and stereoisomers. Should the isomer of the invention be a stereoisomer, the individual stereoisomers (enantiomers and diastereoisomers) and mixtures thereof are included within the scope of the invention. Some of the compounds according to the invention may exist in tautomeric forms (a type of structural isomer), which are also included within the scope of the invention.

By "metabolite" as used herein, it is meant any compound that is an intermediate and/or a product of metabolism. A metabolite from a chemical compound is usually formed as part of the natural biochemical process of degrading and eliminating the compound of interest in a subject to which it is administered. Examples of metabolites of antidepressant agents according to the invention are provided further below.

The term "pharmaceutically or cosmetically acceptable salt" or "salt as used herein refers to a salt that is physiologically tolerated (i.e. non-toxic) when used in an appropriate manner in the context of the present invention, particularly when used on mammals. Pharmaceutically or cosmetically acceptable salts include those derived from pharmaceutically or cosmetically acceptable inorganic and organic acids and bases. Examples of suitable acids according to the invention include hydrochloric, hydrobromic, sulfuric, nitric, perchloric, fumaric, maleic, phosphoric, glycolic, lactic, salicylic, succinic, toluene-p-sulfuric, tartaric, acetic, citric, methanesulfonic, formic, benzoic, malonic, naphthalene-2-sulfuric, benzenesulfonic, gluconic, glutamic, bis-methylenesalicylic, ethanedisulfonic, propionic, p-amino-benzoic, malic, mandelic, cinnamic, citraconic, aspartic, stearic, palmitic, itaconic and sulfamic acids, as well as theophylline acetic acids and 8-halotheophyllines such as the 8-bromotheophylline. Other acids, such as oxalic acid, while not themselves pharmaceutically or cosmetically acceptable, may be employed in the preparation of salts useful as intermediates in obtaining the compounds and their pharmaceutically or cosmetically acceptable acid addition salts. Salts derived from appropriate bases include alkali metal (e.g., sodium), alkaline earth metal, (e.g., magnesium), ammonium and N-(C1-C4 alkyl)₄⁺ salts.

The term "solvate" according to the invention should be understood as meaning any form of the active agent in accordance with the invention (i.e. 5-HT1 BR-stimulating agent), in which said compound is linked through non-covalent interactions to another molecule (normally a polar solvent), including especially hydrates and alcoholates, such as methanolate. Methods of solvation are well-known in the art.

By "clathrate", it is meant herein a chemical substance consisting of a lattice or cage that entraps or contains a second type of molecule/compound of interest, and which can be used to increase the stability and solubility in water of the molecule/compound of interest. Clathrates are typically polymeric.

The term "polymorphs" means herein different crystalline forms of a compound of interest in which molecules have different arrangements and/or different molecular conformation. It includes crystalline liquid form or crystalline solid form of a compound of interest. Hydrates and clathrates can be polymorphs.

By "co-crystal", it is meant herein a crystalline structure composed of at least two components, where the components may be atoms, ions or molecules. Solvates and clathrates may be co-crystals in certain conditions.

In the context of the present invention, the pharmaceutically or cosmetically acceptable derivatives, analogs, isomers, metabolites, salts, solvates, clathrates, polymorphs, and co-crystals as defined above are active, i.e. they exhibit a 5-HT1 BR-stimulating activity. Said activity can be assessed by measuring the increase in cyclic AMP related to 5-HT1 BR or the activation of the MAK kinases as described in Hinton JM et al. 2000. The activation of 5HT1 BR receptor can also be measured via the activation of NFkB (translocation in the nucleus and transcription of its target genes), as described in Chabbi-Achengli Y et al. (2012) or by measuring the activation of pertussis toxin-sensitive G proteins and more generally G protein activations (Pauwels PJ et al, 1998).

It shall further be understood that the 5-HT1 BR-stimulating agents as described herein, or their derivatives, analogs, isomers, metabolites, salts, solvates, clathrates, polymorphs, and co-crystals are preferably in pharmaceutically or cosmetically acceptable or substantially pure form. By pharmaceutically or cosmetically acceptable form, it is meant, *inter alia*, having a pharmaceutically or cosmetically acceptable level of purity, i.e. excluding normal pharmaceutical or cosmetic additives, such as diluents and carriers, and any material considered toxic at normal dosage levels. In the context of the present invention, purity levels are preferably above 98%, more preferably above 99%, and even more preferably above 99.9%. In a preferred embodiment, said purity level is 99.9%.

As stated above, the 5-HT1 BR-stimulating agents according to the invention can be selected among antimigraine drugs, such as triptans or ergotamine. Triptans are well-known in the art as tryptamine-based drugs used in the treatment of migraines and cluster headaches, thanks to their agonistic effects on 5-HT1 BR and 5-HT1 DR. Examples of triptans according to the invention include, but are not limited to, sumatriptan, rizatriptan, zolmitriptan, eletriptan, almotriptan, frovatriptan, naratriptan, avitriptan, and donitriptan. Non limitative examples of salts of said compounds are donitriptan hydrochloride, eletriptan hydrobromide, and rizatriptan benzoate.

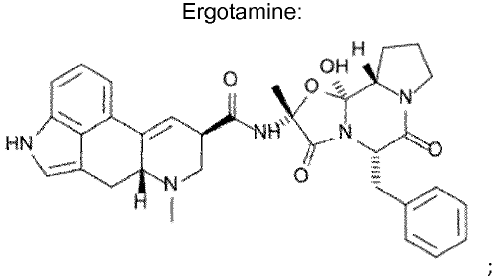

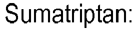

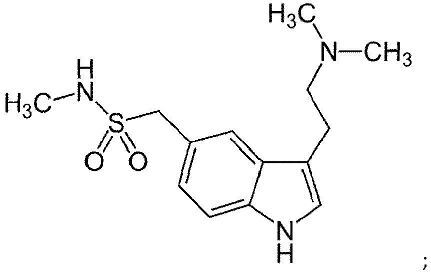

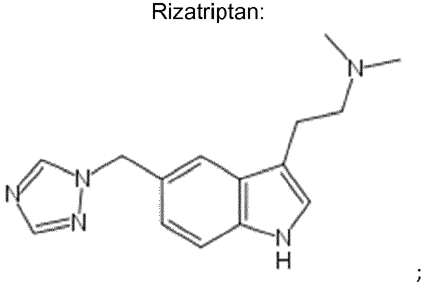

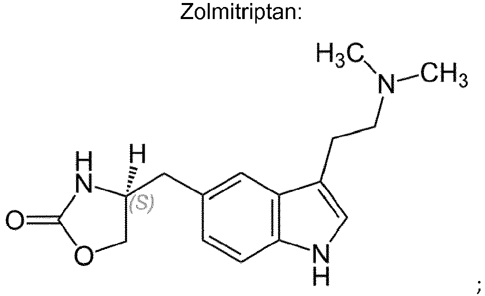

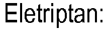

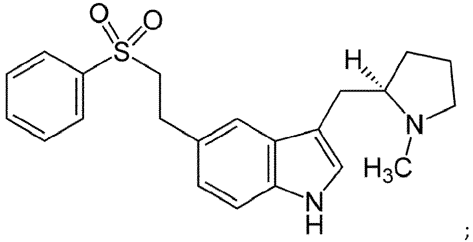

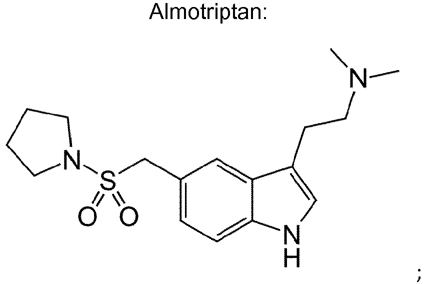

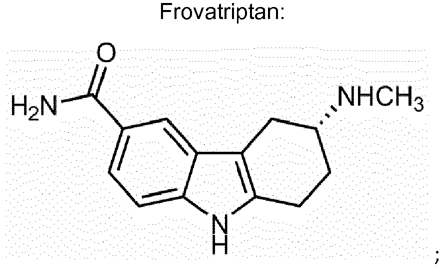

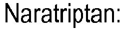

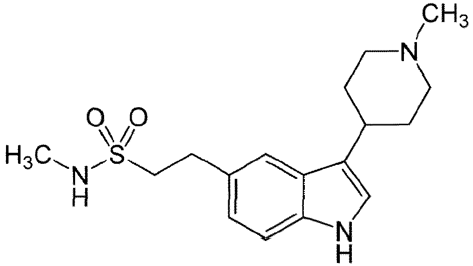

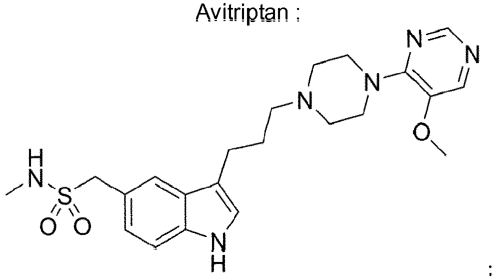

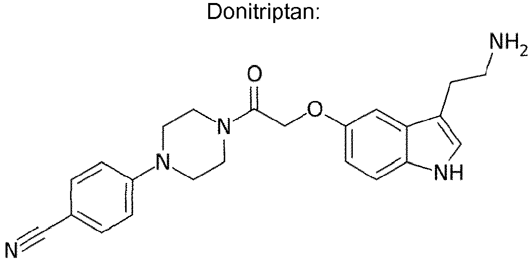

Particularly preferred triptans according to the invention are selected from the group consisting of sumatriptan, rizatriptan, zolmitriptan, eletriptan, almotriptan, and frovatriptan.

The 5-HT1 BR-stimulating agents according to the invention can alternatively be selected among antidepressant agents, as far as they are direct 5-HT1 stimulating agents in accordance with the invention.

By "antidepressant agent", it is meant herein an active agent that is capable to treat mood disorders, such as depression (including severe depression) and/or dysthymia. Antidepressant agents according to the invention include, without limitation, serotonin reuptake inhibitors (SRIs); tricyclic antidepressants (TCAs); monoamine oxidase inhibitors (MAOs); noradrenergic and specific serotoninergic antidepressants (NaSSAs); atypical antidepressants or antidepressant enhancers.

Serotonin reuptake inhibitors (SRIs) designate a class of compounds that typically act by inhibiting the reuptake of the serotonin neurotransmitter into the presynaptic terminal, thereby increasing the serotonin extracellular level and thus serotoninergic transmission. Such compounds can act selectively or non-selectively on the neurotransmitter serotonin. SRIs can indeed also display various degrees of selectivity towards the other monoamine reuptake systems, in particular the transporters for norepinephrine and dopamine. SRIs typically include selective serotonin reuptake inhibitors (SSRIs), serotonine and norepinephrine reuptake inhibitors (SNRIs) and serotonin-norepinephrine-dopamine reuptake inhibitor (SNDRIs).

Examples of selective serotonin reuptake inhibitors (SSRIs) include, without limitation, fluoxetine, citalopram, escitalopram, sertraline, norsertraline, paroxetine, fluvoxamine, femoxetine, indalpine, alaproclate, cericlamine, ifoxetine, zimelidine dapoxetine, and etoperidone, preferably fluoxetine, citalopram, escitalopram, sertraline, norsertraline, paroxetine, fluvoxamine, femoxetine, indalpine, alaproclate, cericlamine, ifoxetine, and zimelidine. As stated above, paroxetine is a SSRI that is preferably not encompassed by the present invention.

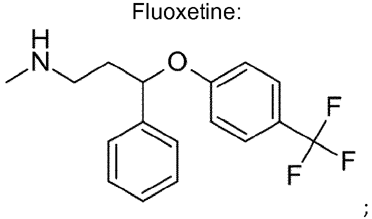

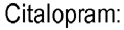

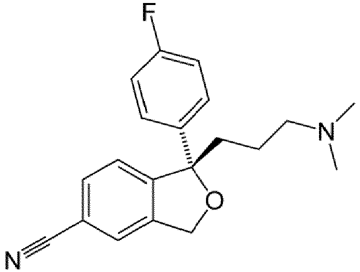

Examples of active SSRIs metabolites include, without limitation, desmethylcitalopram, didesmethylcitalopram, and seproxetine (i.e. (S)-norfluoxetine).

Examples of serotonine and norepinephrine reuptake inhibitors (SNRIs) include, without limitation, duloxetine, venlafaxine, desvenlafaxine, milnacipran, levominalcipran, and sibutramine.

Examples of serotonin-norepinephrine-dopamine reuptake inhibitor (SNDRIs) (also known as triple reuptake inhibitor or TRI) include, without limitation, bicifadine, brasofensine, tesofensine and nomifensine, preferably bicifadine.

Examples of tricyclic antidepressants (TCAs) according to the invention include, without limitation, clomipramine, amoxapine, nortriptyline, maprotiline, trimipramine, imipramine, desipramine and protriptyline.

Examples of monoamine oxidase inhibitors (MAOs) according to the invention include, without limitation, iproniazide, phenelzine, tranylcipromine, moclobemide, selegiline and rasagiline.

Examples of noradrenergic and specific serotoninergic antidepressants (NaSSAs), acting preferably by blocking presynaptic alpha-2 adrenergic receptors, include, among others, mirtazapine, mianserin, aptazapine, esmirtazapine, setiptiline and S32212 (also known as N-[4-methoxy-3-(4-methylpiperazin-1-yl)phenyl]-1,2-dihydro-3H-benzo[e]indole-3-carbo-xamide), preferably mirtazaine and mianserin.

Examples of atypical antidepressants (defined as such as they do not belong to any of the foregoing classes of antidepressants) or antidepressant enhancers include, without limitation, bisarylsulfanyl amines such as vortioxetine, as well as tianeptine, agomelatine, nefazodone, trazodone, buspirone, tandospirone, and ketamine, preferably vortioxetine, tianeptine, agomelatine, nefazodone, trazodone, buspirone, tandospirone, and ketamine.

Bisarylsulfanyl amines have been disclosed in patent application WO 2003/029232, and are within the scope of the 5-HT1 BR-stimulating agents according to the invention. Said compounds can be described according to the following general formula (A): 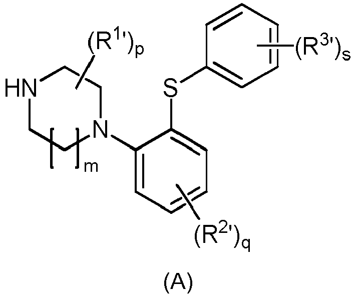 wherein
- m is 1 or 2;
- p is 0, 1, 2, 3, 4, 5, 6, 7 or 8;
- q is 0, 1, 2, 3 or 4;
- s is 1 or 2;
- each R^{1'} is independently selected from the group represented by C₁₋₆-alkyl, or two R^{1'} attached to the same carbon atom may form a 3-6-membered spiro-attached cycloalkyl;
- each R^{2'} is independently selected from the groups represented by halogen, cyano, nitro, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, C₃₋₈-cycloalk(en)yl, C₃₋₈-cycloalk(en)yl-C₁₋₆-alk(en/yn)yl, acyl, C₁₋₆-alk(en/yn)yloxycarbonyl, C₁₋₆-alk(en/yn)ylsulfonyl, or -NR^{x}Ry; -NRxCO-C₁₋₆-alk(en/yn)yl;
- each R^{3'} is independently selected from a group represented by halogen, cyano, nitro, C₁₋₆-alk(en/yn)yl, C₁₋₆-alk(en/yn)yloxy, C₁₋₆-alk(en/yn)ylsulfanyl, hydroxy, hydroxy-C₁₋₆-alk(en/yn)yl, halo-C₁₋₆-alk(en/yn)yl, halo-C₁₋₆-alk(en/yn)yloxy, C₃₋₈-cycloalk(en)yl, C₃₋₈-cycloalk(en)yl-C₁₋₆-alk(en/yn)yl, Ci-6-alk(en/yn)ylsulfonyl, aryl, C₁₋₆-alk(en/yn)yloxycarbonyl, acyl, -NR^{x}CO-C₁₋₆-alk(en/yn)yl, CONR^{x}R^{y} or NR^{x}R^{y};
   or two adjacent R^{3'} substituents together form a heterocycle fused to the phenyl ring selected from the group consisting of 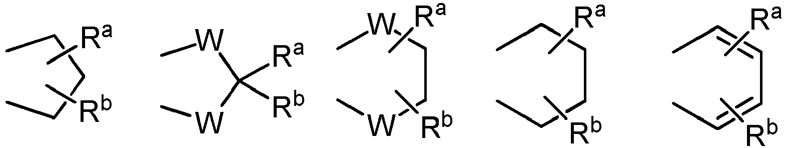
   wherein W is O or S, and R^{a} and R^{b} are hydrogen or C₁₋₆-alkyl; or two adjacent R^{3'} substituents together form a fused heteroaromatic system containing one, two or three heteroatoms,
   wherein each R^{x} and R^{y} is independently selected from the group represented by hydrogen, C₁₋₆-alk(en/yn)yl, C₃₋₈-cycloalk(en)yl, C₃₋₈-cycloalk(en)yl-C₁₋₆-alk(en/yn)yl, or aryl; or R^{x} and R^{y} together with the nitrogen to which they are attached form a 3-7-membered ring which optionally contains one further heteroatom;
   or a pharmaceutically or cosmetically acceptable salt thereof.

Synthesis of compounds of general formula (A) is fully described in WO 2003/029232 and therefore does not need to be detailed herein.

A preferred embodiment of general formula (A) is wherein p is 0.

A preferred embodiment of general formula (A) is wherein m is 1 or 2.

A preferred embodiment of general formula (A) is wherein R^{2'} is trifluoromethyl, or C₁₋₆- alkyl.

A preferred embodiment of general formula (A) is wherein R^{3'} is selected from the group consisting of halogen, C₁₋₆-alkoxy, C₁₋₆-sulfanyl, C₁₋₆- alkyl hydroxy and trifluoromethyl.

A more preferred embodiment of general formula (A) is wherein m=1, p=0, q=0, R^{3'} is methyl and s=2.

Particularly preferred embodiment of general formula (A) is wherein the compound of formula (A) is any of the following:
1-[2-(2-Trifluoromethylphenylsulfanyl)phenyl]piperazine,
1-[2-(4-Bromophenylsulfanyl)phenyl]piperazine,
1-{2-[4-(Methylsulfanyl)phenylsulfanyl]phenyl}piperazine,
1-[2-(4-Hydroxyphenylsulfanyl)]phenyl]piperazine,
1-[2-(2,4-Dimethylphenylsulfanyl)phenyl]piperazine, also known as vortioxetine 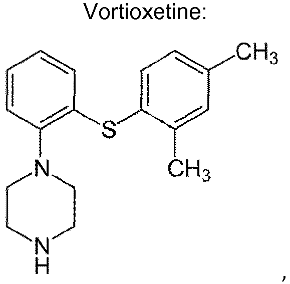
1-[2-(3,5-Dimethylphenylsulfanyl)phenyl]piperazine,
1-[2-(2,6-Dimethylphenylsulfanyl)phenyl]piperazine,
1-[2-(2,5-Dimethylphenylsulfanyl)phenyl]piperazine,
1-[2-(2-Trifluoromethylphenylsulfanyl)phenyl][1,4]diazepane,
1 -[2-(3-Methylphenylsulfanyl)phenyl]-[1,4]-diazepane,
2-(4-Methylphenylsulfanyl)phenyl-1-piperazine,
1-[2-(4-Chlorophenylsulfanyl)phenyl]-piperazine,
1-[2-(4-Methoxyphenylsulfanyl)-4-chlorophenyl]piperazine,
1-[2-(4-Methoxyphenylsulfanyl)-4-methylphenyl]piperazine,
1-[2-(4-Methoxyphenylsulfanyl)-5-methylphenyl]piperazine,
1-[2-(4-Fluorophenylsulfanyl)-5-methylphenyl]piperazine,
1-[2-(4-Methoxyphenylsulfanyl)-5-trifluoromethylphenyl]piperazine,
1-[2-(4-Chlorophenylsulfanyl)phenyl]-3-methylpiperazine,
1-[2-(4-Chlorophenylsulfanyl)phenyl]-3,5-dimethylpiperazine,
or a pharmaceutically or cosmetically acceptable salt thereof.

Most preferred embodiment is wherein the compound of formula (A) is 1-[2-(2,4-Dimethylphenylsulfanyl)phenyl]piperazine (i.e. vortioxetine).

"Halogen" means herein fluoro (F), chloro (CI), bromo (Br) or iodo (I).

"Alkyl", "alkenyl", "alkynyl", and "aryl" are further defined below.

The expression C₁₋₆-alk(en/yn)yl means a C₁₋₆-alkyl, C₂₋₆-alkenyl or a C₂₋₆-alkynyl group.

The expression C₃₋₈-cycloalk(en)yl means a C₃₋₈-cycloalkyl- or cycloalkenyl group.

The term C₁₋₆ alkyl refers to a branched or unbranched alkyl group having from one to six carbon atoms including but not limited to methyl, ethyl, 1-propyl, 2-propyl, 1-butyl, 2-butyl, 2-methyl-2-propyl and 2-methyl-1-propyl.

Similarly, C₂₋₆ alkenyl and C₂₋₆ alkynyl, respectively, designate such groups having from two to six carbon atoms, including one double bond and one triple bond respectively, including but not limited to ethenyl, propenyl, butenyl, ethynyl, propynyl and butynyl.

The term C₃₋₈ cycloalkyl designates a monocyclic or bicyclic carbocycle having three to eight C-atoms, including but not limited to cyclopropyl, cyclopentyl, cyclohexyl, etc.

The term C₃₋₈ cycloalkenyl designates a monocyclic or bicyclic carbocycle having three to eight C-atoms and including one double bond.

In the term C₃₋₈-cycloalk(en)yl-C₁₋₆-alk(en/yn)yl, C₃₋₈-cycloalk(en)yl and C₁₋₆-alk(en/yn)yl are as defined above.

The terms C₁₋₆-alk(en/yn)yloxy, C₁₋₆ alk(en/yn)ylsulfanyl, hydroxy-C₁₋₆-alk(en/yn)yl, halo-C₁₋₆-alk(en/yn)yl, halo-C₁₋₆-alk(en/yn)yloxy, C₁₋₆-alk(en/yn)ylsulfonyl etc. designate such groups in which the C₁₋₆-alk(en/yn)yl are as defined above.

As used herein, the term C₁₋₆-alk(en/yn)yloxycarbonyl refers to groups of the formula C₁₋₆-alk(en/yn)yl -O-CO-, wherein C₁₋₆-alk(en/yn)yl are as defined above.

As used herein, the term acyl refers to formyl, C₁₋₆-alk(en/yn)ylcarbonyl, arylcarbonyl, aryl-C₁₋₆-alk(en/yn)ylcarbonyl, C₃₋₈-cycloalk(en)ylcarbonyl or a C₃₋₈-cycloalk(en)yl-C₁₋₆-alk(en/yn)yl-carbonyl group.

The term 3-7-membered ring optionally containing one further heteroatom as used herein refers to ring systems such as 1-morpholinyl, 1-piperidinyl, 1-azepinyl, 1-piperazinyl, 1-homopiperazinyl, 1-imidazolyl, 1-pyrrolyl or pyrazolyl, all of which may be further substituted with C₁₋₆-alkyl.

The heterocycles formed by two adjacent R^{3'} substituents and fused to the parent ring may together form rings such as 5-membered monocyclic rings such as 3H-1,2,3-oxathiazole, 1,3,2-oxathiazole, 1,3,2-dioxazole, 3H-1,2,3-dithiazote, 1,3,2-dithiazole, 1,2,3-oxadiazole, 1,2,3-thiadiazole, 1*H*-1,2,3-triazole, isoxazole, oxazole, isothiazole, thiazole, 1*H*-imidazole, 1*H*-pyrazole, 1*H*-pyrrole, furan or thiophene and 6-membered monocyclic rings such as 1,2,3-oxathiazine, 1,2,4-oxathiazine, 1,2,5-oxathiazine, 1,4,2-oxathiazine, 1,4,3-oxathiazine, 1,2,3-dioxazine, 1,2,4-dioxazine, 4*H*-1,3,2-dioxazine, 1,4,2-dioxazine, 2*H*-1,5,2-dioxazine, 1,2,3-dithiazine, 1,2,4-dithiazine, 4*H*-1,3,2-dithiazine, 1,4,2-dithiazine, 2H- 1,5,2-dithiazine, 2H- 1,2,3-oxadiazine, 2H- 1,2,4-oxadiazine, 2*H*-1,2,5-oxadiazine, 2H-1,2,b-oxadiazine, 2*H*-1,3,4-oxadiazine, 2*H*-1,2,3-thiadiazine, 2*H*-1,2,4-thiadiazine, 2H-1,2,5-thiadiazine, 2*H*-1,2,6-thiadiazine, 2*H*-1,3,4-thiadiazine, 1,2,3-triazine, 1,2,4-triazine, 2*H*-1,2-oxazine, 2*H*-1,3-oxazine, 2*H*-1,4-oxazine, 2*H*-1,2-thiazine, 2*H*-1,3-thiazine, 2*H*-1,4-thiazine, pyrazine, pyridazine, pyrimidine, 4*H*-1,3-oxathiin, 1,4-oxathiin, 4*H*-1,3-dioxin, 1,4-dioxin, 4H-1,3-dithiin, 1,4-dithiin, pyridine, 2H-pyran or 2*H*-thiin.

Further, the compounds of general formula (A) may exist in unsolvated as well as in solvated forms with pharmaceutically or cosmetically acceptable solvents such as water, ethanol and the like.

Some of the compounds of general formula (A) contain chiral centers and such compounds exist in the form of isomers (i.e. enantiomers). Such isomers and any mixtures thereof including racemic mixtures are also within the scope of the invention.

Particularly preferred antidepressant agents according to the disclosure are selected from the group consisting of fluoxetine, bisarylsulfanyl amines as described above such as vortioxetine, and citalopram, escitalopram, sertraline, paroxetine, fluvoxamine, femoxetine, indalpine, alaproclate, zimelidine, duloxetine, venlafaxine, desvenlafaxine, milnacipran, levominalcipran, sibutramine, bicifadine, clomipramine, amoxapine, maprotiline, imipramine, desipramine, moclobemide, selegiline, mirtazapine, mianserin, tianeptine, agomelatine, trazodone, buspirone, tandospirone, and ketamine. Paroxetine is preferably not encompassed among these preferred antidepressant agents.

More preferably, antidepressant agents according to the disclosure are selected from the group consisting of fluoxetine and bisarylsulfanyl amines as described above such as vortioxetine. Even more preferably, the 5-HT1 BR-stimulating agents according to the invention are selected from the group consisting of bisarylsulfanyl amines as defined above.

Other suitable 5-HT1 BR-stimulating agents according to the invention can be: anpirtoline hydrochloride, CGS-12066A, CGS 12066B dimaleate, oxymetazoline, 5-carboxamidotryptamine, CP-93129 and salts thereof such as CP-93129 dihydrochloride, CP-94253 and salts thereof such as CP-94253 hydrochloride, CP-122,288, CP-135,807, RU-24969 and salts thereof such as RU-24969 hemisuccinate, ziprasidone, asenapine, 5-nonyloxytryptamine oxalate, pindolol and (S)-(-)-pindolol.

Preferably, the 5-HT1 BR-stimulating agent according to the invention is CP-94253, whose formula is:

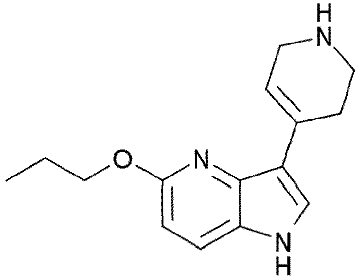

According to a preferred embodiment, the 5-HT1 BR-stimulating agent used in the present invention is an antidepressant selected from the group consisting of SRIs, preferably SSRIs, and atypical antidepressants, preferably atypical antidepressants.

More preferably, the 5-HT1 BR-stimulating agent of the present invention is the atypical antidepressant vortioxetine. Most preferably, the 5-HT1 BR-stimulating agent of the present invention is the atypical antidepressant vortioxetine.

Nevertheless, in the event that the 5-hydroxytryptamine 1B receptor (5-HT1 BR)-stimulating agent of the present invention exerts undesired CNS-related adverse effects, it is particularly advantageous to limit the effects of said agent onto the peripheral serotonin system. Antidepressant agents are notably well-known for exerting such side effects. Side effects can be prevented by chemically modifying the structure of said agent, and by notably grafting a charged chemical moiety to prevent crossing of the blood-brain barrier.

Accordingly, in a preferred embodiment, the 5-HT1 BR-stimulating agent used in the present invention is modified to comprise at least one charged chemical moiety, preferably positively charged. Notably, the positive charge can be retained at a wide range of pH, in particular at a physiological pH.

In other terms, such modified 5-HT1 BR-stimulating agents according to the invention are not capable of crossing the blood-brain barrier. Antidepressant agents and anti-migraine drugs modified in this manner are thus, respectively, anti-depressant disabled and anti-migraine disabled.

Such chemical modifications have been extensively described in patent application WO 2007/148341, and can be performed so as to retain the 5-HT1 BR-stimulating activity of the compounds, while preventing them from crossing the blood-brain barrier.

The term "charged chemical moiety", "charged moiety", "charged chemical group" or "charged group", as used herein, refers to an atom or a group of atoms which forms a part of an organic molecule, and which is characterized by a positive or negative electrostatic charge.

By "positively charged chemical moiety", "positively charged moiety", "positively charged chemical group" or "positively charged group", it is thus meant herein a charged chemical moiety as defined above, which is characterized by a positive electrostatic charge. Compounds which include one or more positively charged moieties are molecular ions often referred to as molecular cations. A positively charged group of atoms has at least one electron less than the number of protons in these atoms. Positively charged chemical moieties include, without limitation, ammonium and sulfonium groups.

A positively charged group which retains its charge at physiological pH is a group that is not capable of participating in proton-exchange interactions at a pH range which is typical to the physiological environment in the body where the 5-HT1-BR stimulating agent is active. Typically, the physiological pH is about 7.4; therefore a positively charged group which retains its charge at physiological pH refers to a positively charged chemical group that stays ionized in a pH range of about 5-8. It is noted that even in the GI, where the pH level is extremely low in terms of physiological pH, the positively charged chemical moiety according to the invention remains positively charged, and hence modified 5-HT1-BR stimulating agents according to the present invention, are not adversely affected by the GI pH levels. Still, yet, according to a further preferred embodiment, said positively charged chemical moiety is a quaternary ammonium group or a tertiary sulfonium group.

By "quaternary ammonium", it is meant herein a nitrogen atom which forms a part of a molecule (an amine) that is attached to four non-hydrogen substituents and thus is positively charged. The term "amine" describes a -NR'R" group where each of R' and R" is independently hydrogen, alkyl, cycloalkyl, heteroalicyclic, aryl or heteroaryl.

By "tertiary sulfonium group", it is meant herein a sulfur atom which forms a part of a molecule (a sulfonium) that is attached to three non-hydrogen substituents and thus is positively charged. The term "sulfonium" refers to a -S⁺R'R", wherein R' and R" are each independently alkyl, cycloalkyl, heteroalicyclic, aryl or heteroaryl.

According to the invention, the term "alkyl group" refers to a linear or branched saturated aliphatic group. Preferably, the alkyl group has 1 to 20 carbon atoms, more preferably 1-10 carbon atoms, and even more preferably between 1-6 carbon atoms. Whenever a numerical range; e.g., "1-10", is stated herein, it implies that the group, in this case the alkyl group, may contain 1 carbon atom, 2 carbon atoms, 3 carbon atoms, etc., up to and including 10 carbon atoms. Examples of alkyl groups include, without limitation, methyl, ethyl, propyl, butyl, tert-butyl and isopropyl groups. The alkyl group can be further substituted. When substituted, the substituent can be, for example, an alkyl, an alkenyl, an alkynyl, a cycloalkyl, an aryl, a heteroaryl, a halide, a hydroxy, an alkoxy and a hydroxyalkyl. The term "alkyl", as used herein, further encompasses saturated or unsaturated hydrocarbon, hence this term further encompasses alkenyl and alkynyl.

The term "cycloalkyl" refers to an aliphatic monocyclic or bicyclic ring having 3 to 8 carbon atoms, and includes, without limitation cyclopropyl, cyclopentyl, cyclohexyl, etc.

The term "alkenyl" describes an unsaturated alkyl, as defined herein, having at least two carbon atoms and at least one carbon-carbon double bond, and which can be substituted by one or more substituents, as described above. The term "alkynyl" is an unsaturated alkyl having at least two carbon atoms and at least one carbon-carbon triple bond, and which can be substituted by one or more substituents, as described above.

The term "aryl" describes an all-carbon monocyclic or fused-ring polycyclic (i.e., rings which share adjacent pairs of carbon atoms) groups having a completely conjugated pi-electron system.

By "aryl or heteroaryl group", it is meant herein a mono- or polycyclic aromatic group comprising preferably between 4 and 15 carbon atoms, preferably between 5 and 10 carbon atoms. Examples of aryl groups include, without limitation, phenyl, naphtyl, etc. The aryl group according to the invention may be further substituted by one or more substituents, as described above. Heteroaryl groups typically comprise at least one heteroatom, such as nitrogen, oxygen, and sulfur - a heteroatom being any atom that is not carbon or hydrogen. Examples of heteroaryl groups include, without limitation, pyrrole, furane, thiophene, imidazole, oxazole, thiazole, pyrazole, pyridine, pyrimidine, quinoline, isoquinoline and purine. The heteroaryl group may be further substituted by one or more substituents, as described above; representative examples are thiadiazole, pyridine, pyrrole, oxazole, indole, purine and the like.

In a more preferred embodiment, said quaternary ammonium group has the formula (I)

-(NR₁R₂R₃)⁺Z⁻ (I)

wherein
Z is an organic or inorganic anion, such as NO₃⁻, H₂PO₄²⁻, Br-, HSO₄⁻, CH₃SO₃⁻, or tartaric acid anion; and
R₁, R₂ and R₃ are each independently selected from the group consisting of alkyl, aryl and cycloalkyl.

Preferably, R₁, R₂ and R₃ are each an alkyl having from 1 to 4 carbon atoms, and more preferably, R₁, R₂ and R₃ are each methyl, resulting in the positively charged group, or the quaternary ammonium group -(NMe₃)⁺.

In another preferred embodiment, said tertiary sulfonium group has the formula (II)

-(SR₄R₅)⁺Z⁻ (II)

wherein
Z is an organic or inorganic anion, such as NO₃⁻, H₂PO₄²⁻, Br-, HSO₄⁻, CH₃SO₃⁻, or tartaric acid anion; and
R₄ and R₅ are each independently selected from the group consisting of alkyl, aryl and cycloalkyl.

Preferably, R₄ and R₅ are each an alkyl having from 1 to 4 carbon atoms, and more preferably, R₄ and R₅ are each methyl, resulting in the positively charged group, or the sulfonium -(SMe₂)⁺.

The positively charged group can be formed on the 5-HT1 BR-stimulating agent from an existing group which forms a part of the 5-HT1 BR-stimulating agent, namely, by turning a partially charged or uncharged group into a positively charged group, or by turning an existing positively charged group which can participate in proton-exchange interaction into one that cannot participate in such interaction, making it into an irreversible positive charge, or a permanent positive charge, thereby modifying the 5-HT1 BR-stimulating agent.

Alternatively, the positively charged group can be added to the 5-HT1 BR-stimulating agent by substituting one or more carbon atom with a positively charged group, e.g., by replacing a hydrogen atom or any other substituent with a quaternary ammonium or a tertiary sulfonium group.

Examples of preferred 5-HT1 BR-stimulating agents from which the compounds described herein can be derived include, as far as they aare direct 5-HT1stimulating agents, fluoxetine, bisarylsulfanyl amines as described above such as vortioxetine, as well as citalopram, alaproclate, dapoxetine, fluvoxamine, paroxetine, sertraline, venlafaxine,zimelidine, etoperidone, densvalafaxine, duloxetine, minalcipran, nefazodone, venlafaxine, brasofensine, tesofensine and nomifensine, preferably vortioxetine, fluoxetine, citalopram, alaproclate, dapoxetine, fluvoxamine, paroxetine, sertraline, venlafaxine and zimelidine. Indeed, all these agents already comprise at least one amine group, which can be readily converted into a quaternary ammonium, i.e. a positively charged group as defined above. In particular, said agents can be modified to comprise at least one quaternary ammonium group of formula (I) as described above. As stated above, paroxetine is preferably not encompassed by the present invention.

An example of derivative of citalopram that comprises such a quaternary ammonium group is n-methyl-citalopram (NMC), of which the synthesis is fully detailed in patent application WO2007/128341.

Bisarylsulfanyl amines of formula (A) are also herein particularly advantageous as they comprise not only an amine group, but also a sulfur group, which can be readily converted into a quaternary ammonium group and/or into a tertiary sulfonium group, respectively. Positively charged moieties can also be attached to the carbon atom(s) of the piperazine group of said compounds.

Particularly preferred derivatives of said bisarylsulfanyl amines are compounds of formula (B) as follows: 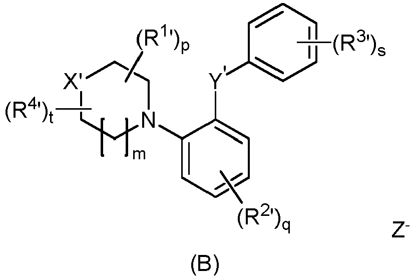 wherein
- Z is an organic or inorganic anion as defined above, such as NO₃⁻, H₂PO₄²⁻, Br-, HSO₄⁻, CH₃SO₃⁻, or tartaric acid anion, or a mixture of organic or inorganic anions, whose global charge is such that the compound of formula (B) is neutral;
- R^{1'}, R^{2'}, R^{3'}, m, p, q and s are as defined above, wherein R^{3'} can optionally be a C₁₋₆-alk(en/yn)yloxy group substituted by an ammonium or sulfonium group as defined above, preferably R^{3'} is choline;
- t is 0, 1, 2, 3, 4, 5, 6, 7 or 8, preferably 0 or 1, more preferably 0, with the proviso that t+p ≤ 8;
- each R^{4'} is at least one charged chemical moiety, identical or different, preferably positively charged, as defined above, such as a sulfonium or an ammonium group;
- X' is selected from the group consisting of:
   ∘ -(NR^{5'}R^{6'})⁺- wherein
      R^{5'} and R^{6'} are each independently selected from the group represented by hydrogen, alkyl, aryl and cycloalkyl as defined herein, preferably by an hydrogen, a C₁₋₆-alkyl, and a C₃₋₈-cycloalkyl ; or
      R^{5'}R^{6'} form together with the nitrogen to which they are attached a cycloheteroalkyl, preferably a 3-8-membered cycloheteroalkyl, more preferably a 3-6-membered cycloheteroalkyl;
   o -NH-;
   o -NR^{7'}-wherein R^{7'} is a C₁₋₆-alkyl;
   ∘ -N⁺(O⁻)R^{8'-} wherein R^{8'} is a C₁₋₆-alkyl;
   o -NC(O)R^{9'}- wherein R^{9'} is an amino acid, said amino-acid being preferably positively charged such as histidine, arginine or lysine, or an amino acid derivative, said derivative being preferably positively charged such as choline or carnitine, or a C₁₋₆-alkyl phosphonium;
- Y' is selected from the group consisting of:
   o -S-;
   ∘ -(SR^{10'})⁺- wherein R^{10'} is selected from the group represented by hydrogen, alkyl, aryl and cycloalkyl as defined herein, preferably is a C₁₋₆-alkyl ; and
   o -S⁺(O)⁻-

The skilled person in the art would readily understand that the anions Z are present to counterbalance the positive charges on the molecule. Accordingly, compounds of formula (B) comprise as many anions Z as necessary to neutralize the positive charges of the molecule. One skilled practitioner would further understand that when p>0 and t>0, R^{1'} and R^{4'} are attached to any of the carbon atoms of the heterocyclic ring, albeit to different carbons.

In a preferred embodiment, only one of X', Y', R^{4'} (when t>0) and R^{3'} (when substituted by an ammonium or sulfonium group) is a positively charged chemical moiety.

Preferred embodiments regarding R^{1'}, R^{2'}, R^{3'}, m, p, q and s are as defined above.

In a preferred embodiment of the invention, the 5-HT1 BR-stimulating agent from which the compounds described herein are derived, to notably preferably comprise a charged chemical moiety, is selected from the group consisting of atypical antidepressants such as bisarylsulfanyl amines as defined above.

More preferably, the 5-HT1 BR-stimulating agent to be modified is the atypical antidepressant vortioxetine.

Most preferably, the 5-HT1 BR-stimulating agent to be modified is vortioxetine.

For example, vortioxetine can be chemically modified, as follows: 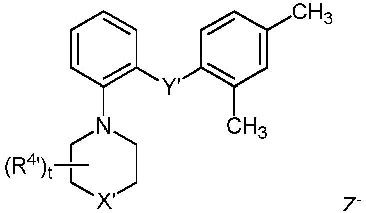 wherein Z, t, R^{4'}, X' and Y' are as defined above. More preferably, t=0.

Particularly preferred salts, derivatives and/or analogs of vortioxetine, which comprise at least one charged chemical moiety, preferably positively charged, are selected from the group consisting of:
- 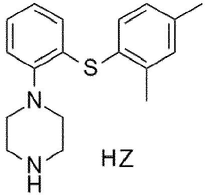 wherein HZ is preferably HNO₃, H₃PO₄, HBr, H₂SO₄, CH₃SO₃H, or tartaric acid (salts of vortioxetine);
- 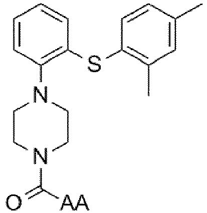 wherein AA is an amino acid, preferably 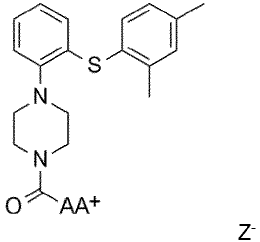 wherein AA+ is a positively charged amino acid such as histidine, arginine or lysine;
- 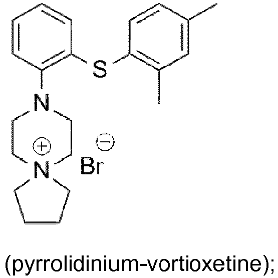
- 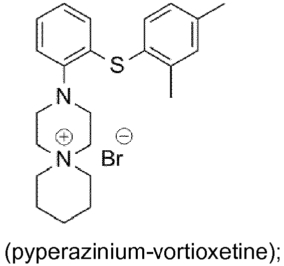
- 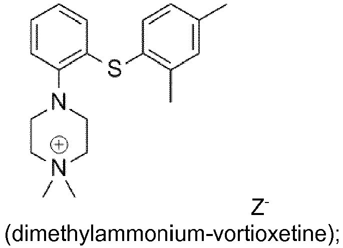
- 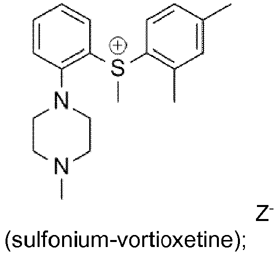
- 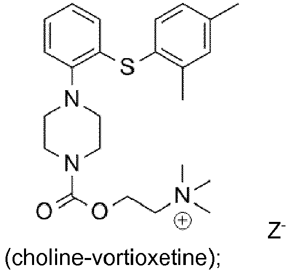
- 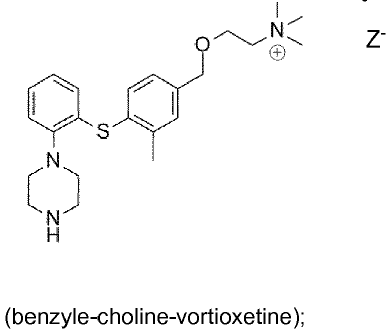
- 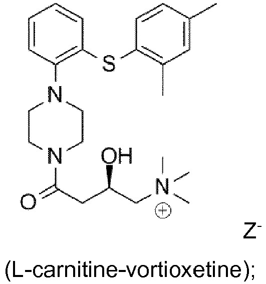
- 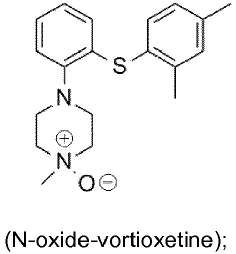
- 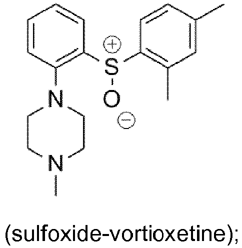
- 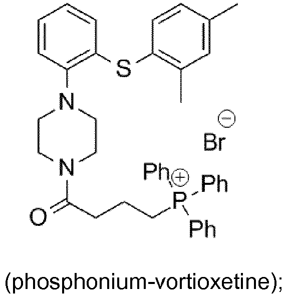 and
-

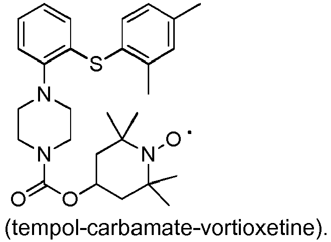

Preferably, said salts, derivatives and/or analogs of vortioxetine, which comprise at least one charged chemical moiety, preferably positively charged, are selected from the group consisting of:
- salts of vortioxetine as described above;
- vortioxetine coupled to a positively charged amino acid (preferably at least one) such as histidine, arginine or lysine, as described above;
- pyrrolidinium-vortioxetine;
- pyperazinium-vortioxetine;
- dimethylammonium-vortioxetine;
- sulfonium-vortioxetine;
- N-oxide-vortioxetine;
- sulfoxide-vortioxetine;
- phosphonium-vortioxetine; and
- tempol-carbamate-vortioxetine.

More preferably, said salts, derivatives and/or analogs of vortioxetine, which comprise at least one positively charged chemical moiety, are selected from the group consisting of:
- salts of vortioxetine as described above;
- vortioxetine coupled to a positively charged amino acid (preferably at least one) such as histidine, arginine or lysine, as described above ;
- pyrrolidinium-vortioxetine;
- pyperazinium-vortioxetine;
- dimethylammonium-vortioxetine;
- sulfonium-vortioxetine;
- N-oxide-vortioxetine;
- sulfoxide-vortioxetine ; and
- phosphonium-vortioxetine.

Yet, even more preferably, said salts, derivatives and/or analogs of vortioxetine, which comprise at least one positively charged chemical moiety, are selected from the group consisting of:
- salts of vortioxetine as described above;
- vortioxetine coupled to a positively charged amino acid (preferably at least one) such as histidine, arginine or lysine, as described above ;
- pyrrolidinium-vortioxetine;
- pyperazinium-vortioxetine;
- dimethylammonium-vortioxetine;
- sulfonium-vortioxetine ; and
- phosphonium-vortioxetine.

Still, even more preferably, said positively charged vortioxetine is selected from the group consisting of histidine-vortioxetine and pyrrolidinium-vortioxetine.

The above compounds can be prepared according to conventional methods in the art. Such methods are described in further details below.

For example, in order to synthetize pyrrolidinium-vortioxetine or pyperazinium-vortioxetine, one skilled person in the art can proceed as follows:

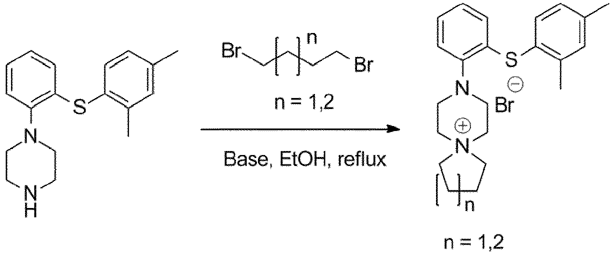

More particularly, for pyrrolidinium (n=1) formation on 4-arylpiperazine, the reaction can be performed using either K₂CO₃, ethanol (EtOH) and reflux for 10h (Mokrosz et al., 1992, incorporated herein by reference), or K₂CO₃, acetone and reflux for 15h (see WO 2004/99114A1).

Still, for example, in order to synthetize dimethylammonium-vortioxetine, one skilled person in the art can proceed as follows:

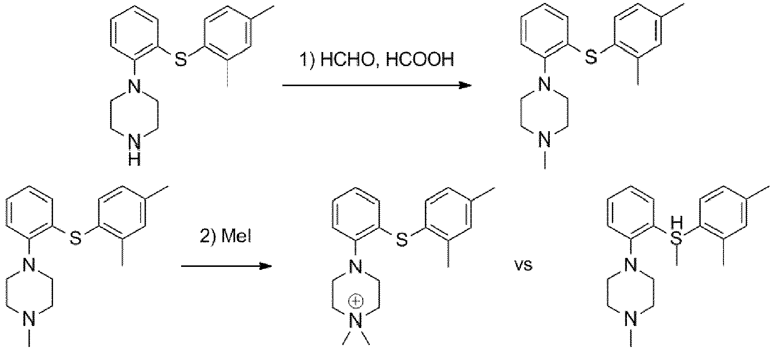

More particularly, for 4-arylpiperazine dimethylation, one can refer to Romanelli et al. (2001) the first step being an Eschweiler-clarke reaction, and the second step being a methylation.

As another illustrative example, in order to synthetize amino acid derivatives of vortioxetine, choline-vortioxetine and carnitine-vortioxetine, one skilled person in the art can proceed as follows:

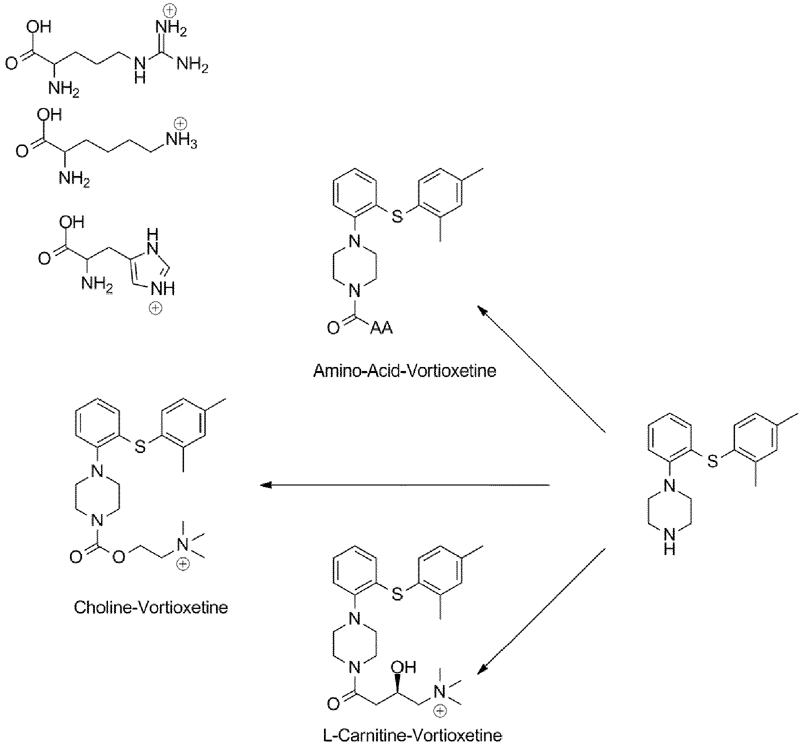

More particularly, for amide formation on the carnitine, the reaction can be performed on either acylhydrazine using pyridine, ethylene dichloride (EDC), dimethylformamide (DMF), and ethanol (EtOH) (Kuroda et al., 1996), or on primary amine using pyridine, ethylene dichloride (EDC), methanol (MeOH) and acetonitrile (CH₃CN) (Nakaya et al., 2001).

As another illustrative example, in order to synthetize phosphonium-vortioxetine, one skilled person in the art can proceed as follows:

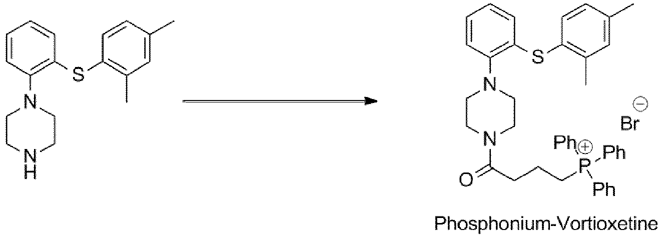

As another illustrative example, in order to synthetize N-oxide-vortioxetine, one skilled person in the art can proceed as follows:

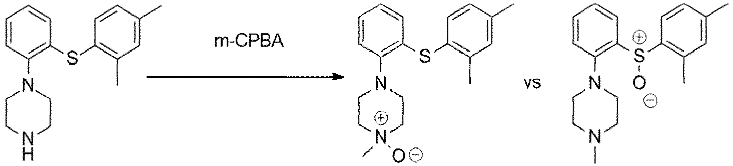

More particularly, for N-oxidation of 4-aryl-piperazines, the reaction can be performed using meta-chloroperoxybenzoic acid (m-CPBA) and CH₂Cl₂ at 20-45°C (see US 2008/153812A1, WO 2011/162515A2 or WO 2004/104007A1).

As another illustrative example, in order to synthetize tempol-carbamate-vortioxetine, one skilled person in the art can proceed as follows:

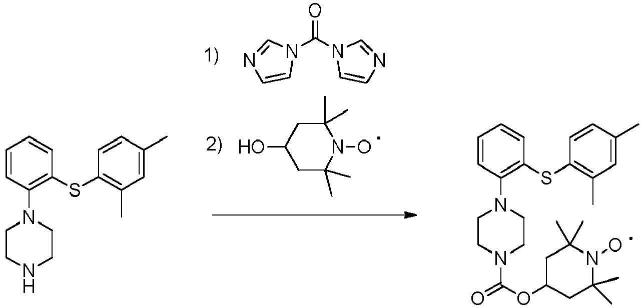

As another illustrative example, in order to synthetize benzyl-choline-vortioxetine, one skilled person in the art can proceed as follows:

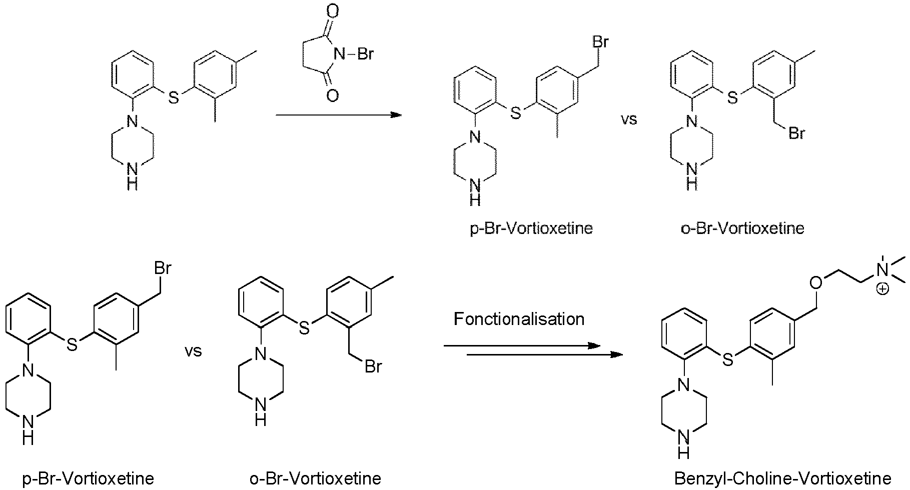

More particularly, for benzylic position bromation, the reaction can be performed using either N-bromosuccinimide, azobisisobutyronitrile (AIBN) and tetrachloromethane (CCl₄) (see US 2010/4159A1, incorporated herein by reference), or N-bromosuccinimide, meta-chloroperoxybenzoic acid (m-CPBA) and tetrachloromethane (CCl₄) (see Farmaco, 1989, 44, from 683).

The present invention will be better understood in the light of the following detailed description of experiments, including examples. Nevertheless, the skilled artisan will appreciate that this detailed description is not limitative and that various modifications, substitutions, omissions, and changes may be made without departing from the scope of the invention.

### BRIEF DESCRIPTION OF THE DRAWINGS

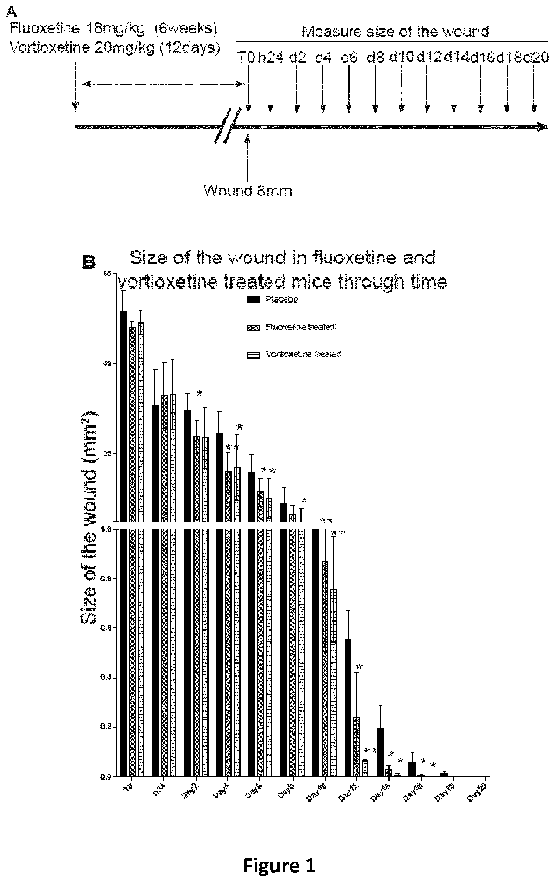

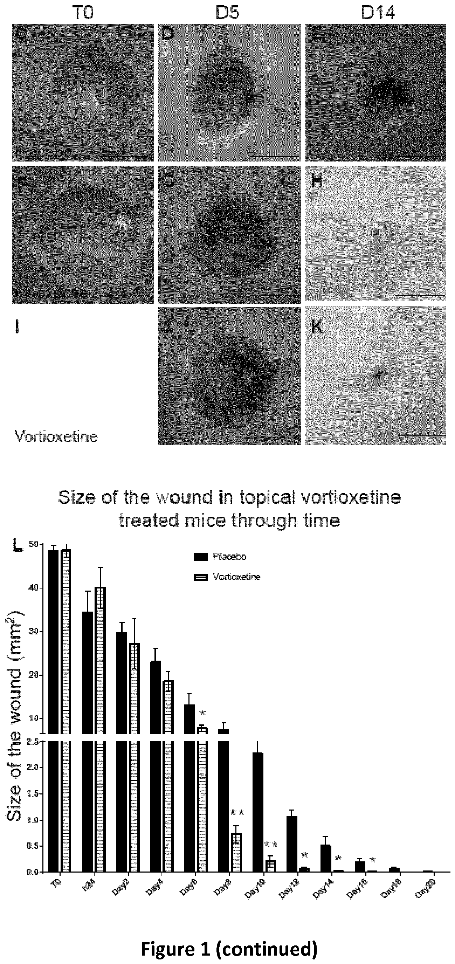

Figure 1: Fluoxetine and vortioxetine speed up the wound healing process. Macroscopic observations. (A) Scheme of fluoxetine and vortioxetine delivery and sampling times. (B) Size of the wound through time in the treated and placebo mice. The treatment was given prior to the injury and continued during the healing process. (C-K) Illustrations of wounds at different time points under different treatments. (L) Size of the wound after injury and topical application of vortioxetine. No pre-treatment was performed. Scale bar is 4mm, * p≤0.05; **p≤0.01; nothing: not significant. All statistics are done with Mann-Whitney test and compared with placebo.

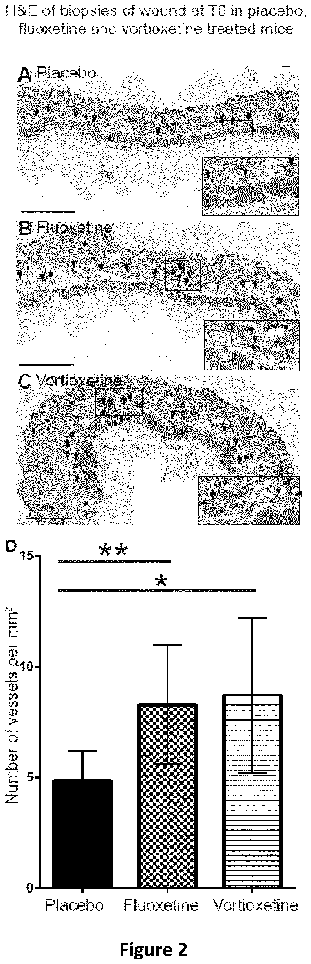

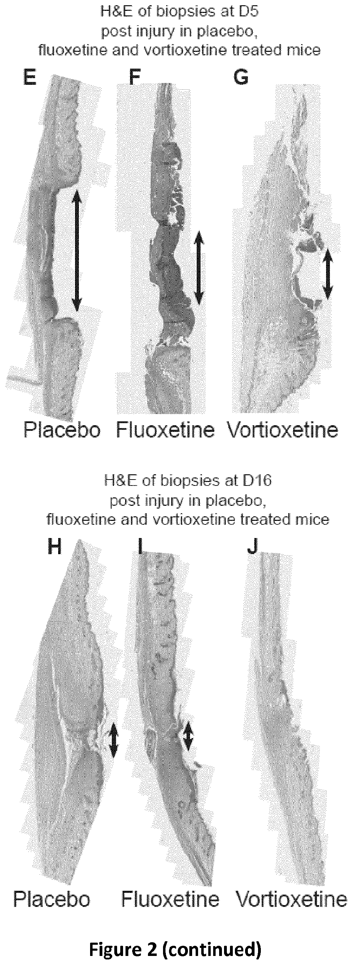

Figure 2: Fluoxetine and vortioxetine speed up the wound healing process. Histological observations. (A-C) H&E staining of the wounds at T0, right after the injury, the biopsies were fixed in formol and H&E stained. Arrows are pointing the vessels. (D) Quantification of the number of vessels per mm² in the placebo, fluoxetine and vortioxetine treated mice. (EG) H&E of skin around the wound 5 days post injury. Arrows represent the size of the wound. (H-J) H&E of skin around the wound 16 days post injury. Arrows represent the size of the wound. Scale bar is 2mm, * p≤0.05; **p≤0.01; nothing: not significant. All statistics are done with Mann-Whitney test and compared with placebo.

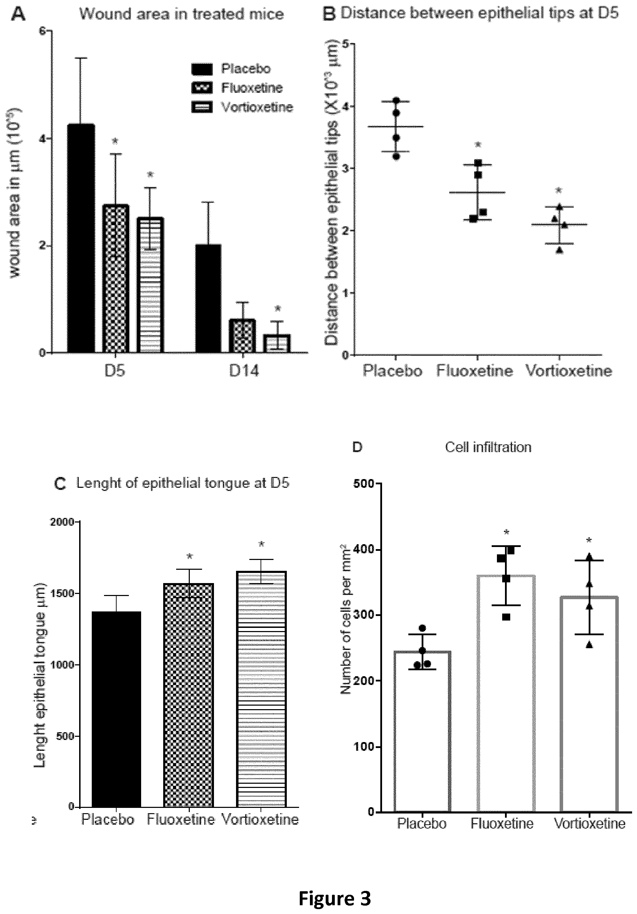

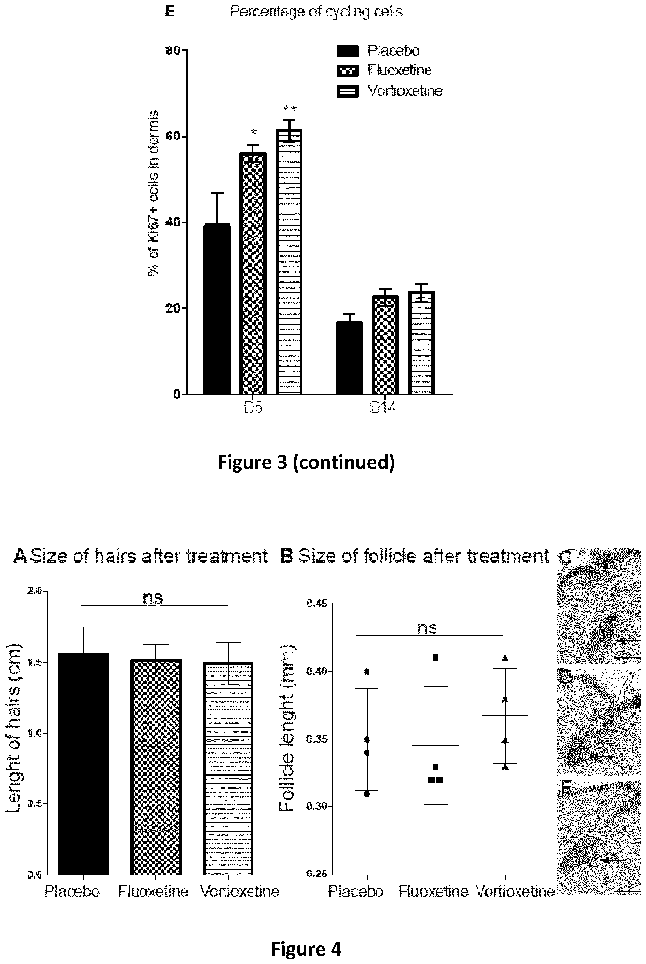

Figure 3: Cellular behaviour at the site of the wound healing after fluoxetine and vortioxetine treatment. (A) Histological measurement of the wound area. (B) Distance between epithelial tips 5 days post injury. (C) Length of the epithelial tongue 5 days post injury. (D) Cell infiltration in the dermis 5 days post injury. (E) Percentage of Ki67+ representing the cycling cells 5 and 14 days post injury in the fluoxetine, vortioxetine and placebo treated mice. * p≤0.05; **p≤0.01; nothing: not significant. All statistics are done with Mann-Whitney test and compared with placebo.

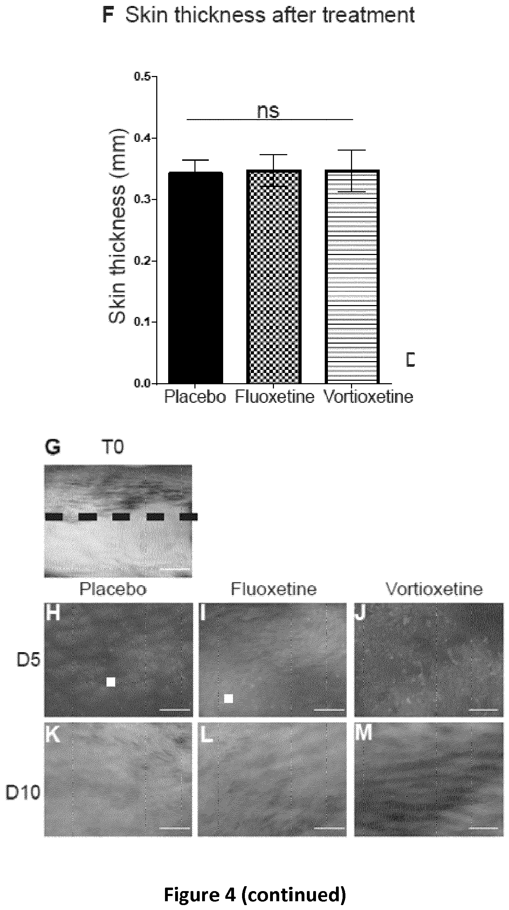

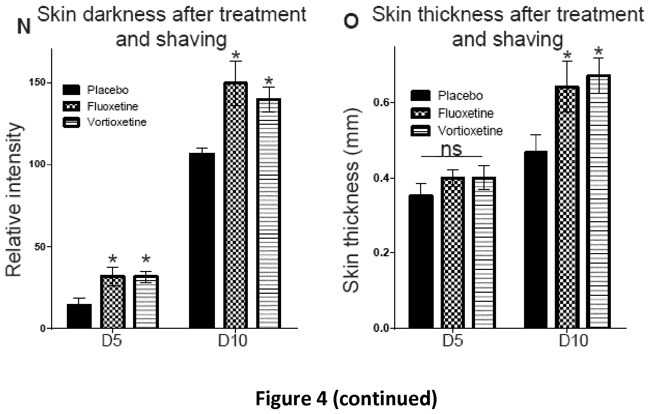

Figure 4: No changes in hair length is detected under treatment but hair growth after shaving is faster. (A) Length of hairs measured one by one (n=50 hairs per mice and n=4 mice) in the placebo, fluoxetine and vortioxetine treated mice. (B) Size of the hair follicle after treatment. (C-E) H&E representing a transversal cut of the skin after treatment. Arrow pointing the hair follicle. (F) Thickness of the skin after placebo, fluoxetine and vortioxetine treatment. (G) Representative picture of the 2 protocol used on the same mouse. Right part is shaved with electric clipper; left part is shaved with wax. (H-M) pictures of the skin of mice in placebo, fluoxetine and vortioxetine treated animals, 5 and 10 days after depilation. (N) Relative intensity of darkness of the skin in the placebo, fluoxetine and vortioxetine treated mice measured after photography and image J relative intensity of luminescence. (O) Skin thickness in placebo, fluoxetine and vortioxetine treated mice 5 and 10 days after shaving.

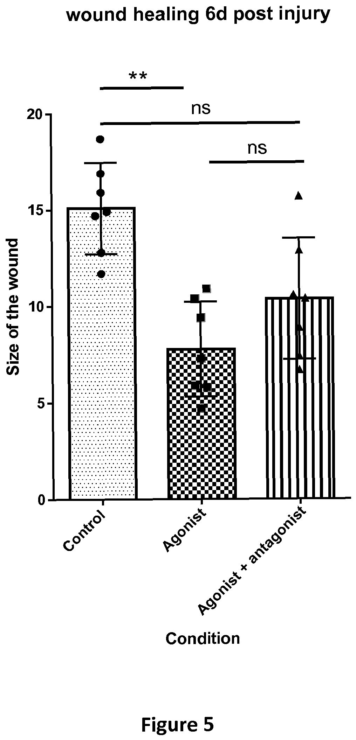

Figure 5: Size of the wound 6 days after injury in control mice, mice topically treated with CP 94253 (0.5µmol) and mice topically treated with the 5HT1 BR agonist CP 94253 and the 5HT1 BR antagonist GR127935 as disclosed in example 3.

### EXAMPLES

### Fluoxetine and vortioxetine accelerate wound healing and hair growth

### 1. MATERIAL AND METHODS

### 1.1. Mice and injections

All procedures in this study were approved by the Animal Care and Use committee at the Institut Pasteur (CETEA 2016-0007). Unless specified, 6 weeks old male mice C57BI/6Rj were used in this study and housed on a 12:12 light/dark cycle in a pathogen free facility with controlled temperature and humidity. Food and drink were given ad libitum. Fluoxetine was dissolved in 0.9% NaCl and delivered either by intra peritoneal injections or per os at 18mg/Kg. Control group received daily injections of 0.9% NaCl. For vortioxetine mice also received the product either dissolved in 96% Ethanol and then diluted 1/200 in water ad libitum at 20mg/kg or dissolved in 96% Ethanol and then diluted 1/200 in 0.9% NaCl, final concentration 20mg/kg body weight corrected. For topical applications vortioxetine was dissolved in 0.9% Nacl and directly applied in the wound the 3 first days and then on top on the wound the following days, every day.

### 1.2. Wound biopsy

Mice were anesthetized using Isoflurane, initially in an induction chamber. The anaesthetised mice were transferred to the anaesthetic nose-cone and maintained on a heated block throughout the procedure (typically for about 5 minutes). Analgesic was administered by subcutaneous injection e.g. buprenorphine 1.5µl of 5% w/v solution, diluted in 30µl sterile water. The area of the biopsy was shaved. Then an area of dorsal skin was rendered sterile by swabbing with Videne surgical scrub followed by sterile water. A 8mm sterile punch biopsy was used to create an incision through the epidermis / dermis to the muscle fascia. Any bleeding at this step was carefully avoided. The punch biopsy was removed and the skin separated from muscle connective tissues using forceps or blunted scissors. Mice were then removed from the anesthetic nose-cone and housed in sterilised cages containing paper liner and paper bedding. They were kept warm (using a cage placed on a heated mat) until fully recovered from the effects of the anaesthetic.

### 1.3. Mice shaving

2 different protocols were used to remove the hair from the mice. Mice were anesthetized using Isoflurane, initially in an induction chamber. The anaesthetised mice were transferred to the anaesthetic nose-cone and maintained on a heated block throughout the procedure (typically for about 5 minutes). In protocol 1, shaving was performed by cutting the hair: the mice were gently restrained manually and half of their back was shaved with portable electric hair clippers. In protocol 2, some hair was firstly removed with forceps and wax was used to unstack the hair. The wax was applied twice for maximum efficiency.

### 1.4. Histological analysis

Tibialis anterior muscle was carefully dissected and snap frozen in liquid-nitrogen-cooled isopentane for a few minutes and stored at -80°C prior to cryosectioning (10µm sections). Sections were kept at room temperature overnight before staining. Sections were then rehydrated in PBS for 10 minutes and fixed in 10% formalin for 3 minutes. The sections were then routinely stained with haematoxylin and eosin (H&E) using an automated stain machine. The slides were assessed by double blinding and automated when possible (fibre diameter, cell count).

### 1.5. Re-epithelization and measure of epithelial tongue

Width of the wound and distance of the traversed epithelium were measured on H&E. Percent of re-epithelization was calculated according to the following formula: [distance covered by epithelium] / [distance between wound bed] X100.

The extent of epithelialization and granulation tissue formation was determined on H&E-stained paraffin tissue sections. The length of the epithelial tongue was determined as the distance that the neo-epithelium had migrated from the margin of the wound as defined by the presence of hair follicles in non-wounded skin.

### 1.6. Skin color

The skin color of the mouse skin was measured three times in 5 different locations of the shaved skin randomly and averaged within a 16X16mm2 field, using ImageJ. The luminance measured reflects the relative darkness ranging from white (0) to black (1 0 0).

### 1.7. Immunostainings

Immunostaining was performed on formol fixed sections. Permeabilized with 0.5% Triton X-100 20 min at room temperature, washed, and blocked with 10% BSA for 30 min. Sections were incubated with primary antibodies overnight at 4°C and with Alexa-conjugated secondary antibodies 1/250 and Hoechst for 45 minutes. Sections were then analysed using an automated axioscan (Zeiss) or inverted Observer.Z1 Apotome (Zeiss).

### 1.8. Statistical analysis

Statistical analysis was performed using GraphPad Prism software using appropriate tests (non-parametric Mann-Whitney unless specified) and a minimum of 95% confidence interval for significance; p values indicated on figures are < 0.05 (*), < 0.01(^{∗∗}), and < 0.001 (^{∗∗∗}). Figures display average values of all animals tested ± SD or ± SEM for RT-qPCR, or as indicated.

### 2. RESULTS

### 2.1. Macroscopic observations of the wound healing

In order to assess the regenerative potential of the skin, a 8mm biopsy of the skin was performed in C57BI/6 mice, which were either treated *per os* with fluoxetine at 18mg/kg or with vortioxetine at 20mg/kg for 6 weeks and 12 days respectively (Figure 1A) and during the healing process.

No difference was noted in the size of the wound for the first 24hours. Two days post injury, a smaller size in the wound of fluoxetine was observed in treated animals compared with placebo (Figure 1B). Four days post injury, the wound was smaller in both fluoxetine and vortioxetine treated animals compared with placebo group (Figure 1B). In the course of wound closure, the size was constantly smaller in fluoxetine and vortioxetine treated animals until 16 days post injury (Figure 1B-K). Six days post injury, the area of the wound was 15.67mm²±4.2 in the placebo, 11.48 mm²±3.1 in the fluoxetine treated and 10.10 mm²±4.4 in the vortioxetine treated animals (Figure 1B, D, G). Sixteen days post injury, the skin of the fluoxetine and vortioxetine treated animals was virtually completely regenerated at the macroscopic level. Indeed the area of the wound was still 0.5mm²±0.03 in the placebo against 0.05mm²±0.004 in the fluoxetine treated animals and vortioxetine (Figure 1B, H, K).

To further confirm the data, the same protocol was performed on C57BI/6 mice wounded with 8mm biopsy, but this time the mice were not pre-treated and received topical (300µl) applications of vortioxetine at a concentration of 20mg/ml diluted in 96% ethanol. The placebo received the same amount of 96% ethanol. No differences between the sizes of the wounds were observed from T0 (when the wound is created) to 4 days post injury. Six days post injury, the size of the wound was smaller in the animals topically treated with vortioxetine (Figure 1L). Eight days post injury, the size of the wound was 10 times lower than the placebo (Figure 1L). Sixteen days post injury, the size of the wound was 0.20mm²±0.07 whereas it was virtually completely closed in the topical vortioxetine treated (Figure 1L).

### 2.2. Histological observations 5 and 14 days post injury

To further characterize the wound closure, histological analysis was performed at the time of the biopsy (T0), 5 days post injury and 14 days post injury.

At the time of the biopsy,the skin was collected from the wound and the number of vessels was counted by H&E staining. Animals treated with fluoxetine or vortioxetine had more vessels than mice that received the placebo (Figure 2A-D). Indeed, the placebo displayed 4.8±1.3 vessels/mm² whereas the fluoxetine-treated mice displayed 8.2±2.7 vessels/mm² and the vortioxetine-treated mice displayed 8.7±3.5 vessels/mm² (Figure 2D).

To further confirm these macroscopic observations, an H&E staining was also performed on the injured skin 5 and 14 days post biopsy. A smaller open wounded area was observed in the fluoxetine and vortioxetine-treated mice both at 5 days post injury (Figure 2E-J). Measurement of the wound area on digital images showed that the differences between fluoxetine and vortioxetine treated mice and placebo mice were statistically significant on day 5 and day 14 post injury (Figure 3A).

Another readout of skin regeneration is the extent of re-epithelization. On H&E-stained paraffin sections, representing the longitudinal diameter of the wound, significantly shorter distances between the tips of the epithelial tongues were measured for the wounds of fluoxetine and vortioxetine mice compared with placebo animals on days 5 wounding (Figure 3B). The length of the epithelial tongue itself was determined as the distance that the neo-epithelium had migrated from the margin of the wound as defined by the presence of hair follicles in non-wounded skin. Likewise, the length of the epithelial tongues was significantly increased in fluoxetine and vortioxetine treated mice in comparison with placebo mice on day 5 after injury (Figure 3C). Interestingly more cells were detected in the fluoxetine and vortioxetine treated mice when compared with placebo treated mice 5 days post injury (Figure 3D). This infiltration was due to higher cell division and immune cell infiltration (inflammation). A first evidence came from the number of KI67+ cells (marker of proliferating cells) that is higher in the wound of fluoxetine and vortioxetine treated mice compared with placebo 5 days post injury (Figure 3E). No differences were detected 14 days post injury in the number of cycling cells (Figure 3E).

### 2.3. Hair growth is correlated to wound healing

The hair follicle (HF) is one of the most functionally diverse mini-organs in the mammalian body, which mediates functions as varied as thermal homeostasis, structural protein and hormone biosynthesis, immune defense, UV protection, sensory perception (Schneider et al., 2009). Over several decades, intensive research has characterized the phenotypical flexibility and immense proliferative capacity of HF cells under conditions of homeostasis. In contrast, although correlation between the HF and skin wound healing/regeneration has long been noted, only recently has the molecular and cellular basis for these observations begun to be addressed. Early evidence revealed that shallow wounds, where portions of HFs remained intact, healed more quickly. Recent studies have shown that a substantial portion of the neoepidermis in wounded skin is provided by epithelial stem cells residing in the HF (Ito et al., 2005; Levy et al., 2007; Snippert et al., 2010). This response seems specific to wounding, as the HF does not appear to contribute to epithelial homeostasis under physiological conditions.

Considering that the present data demonstrate a better regeneration of the skin after a treatment with fluoxetine or vortioxetine, and with the existing strong links between HF and skin regeneration via its stem cells, it was further investigated whether the HF could benefit from such treatment.

To study the possible effect of fluoxetine and vortioxetine on hair growth, fluoxetine (at 18mg/kg) or vortioxetine (at 20mg/kg) was provided peros to mice for 6 weeks and 12 days, respectively. No differences in hair size was detected (Figure 4A), neither in hair follicle length (Figure 4B-E). Last, skin thickness, which has been shown to correspond to hair follicle length and defined as the distance from the epidermal granular layer to the top edge of the *panniculus carnosus,* did not change (Figure 4F). However, when the hairs were challenged with shaving or depilation, a faster growth of hairs was observed

In order to study the influence of fluoxetine or vortioxetine on hair growth after a stress 2 different protocols were used on the same mouse. The half right part of the mouse back was shaved by cutting the hair with hairs clippers, and, in parallel, the hairs were removed using wax to unstack the hairs on the left part of the back (Figure 4G). A faster growth of hairs was observed in both protocols (Figure 4G-O). More advanced hair growth was observed 5 days after shaving in the animals treated with fluoxetine or vortioxetine compared with placebo (Figure 4I,L,N). This was still true 10 days after shaving with longer hairs in the treated animals compared with placebo (Figure 4 J,M,N). However, 24 days after shaving, when hairs were supposed to be fully regenerated, no differences could be noted between the groups. The skin thickness measured by H&E staining confirmed the previous observation of a more advanced, faster regeneration of the hairs after shaving under fluoxetine and vortioxetine treatment (Figure 4O). These observations are in favor of a faster growth and faster regeneration but not bigger or longer hairs.

### 2. DISCUSSION

In this study, the inventors showed that the delivery of either fluoxetine or vortioxetine was speeding up the wound closure of injured mice. Indeed, the wound was in average 27% smaller after fluoxetine treatment and 35% smaller after vortioxetine treatment between the 4^{th} and the 14^{th} day post-injury. The same observation was also made when delivering the vortioxetine directly onto the skin (i.e. topically) without any pre-treatment, however longer delays were observed. Interestingly, when in quiescence (telogen phase) the hair did not seem to react to the treatment as the hair follicle or length of the hairs did not change. However, it seemed that the hair follicle growth was improved after shaving in treated mice compared with placebo.

Although the skin seemed to also be impacted after fluoxetine or vortioxetine treatment alone (without shaving or biopsy), this particular condition needed to be further investigated with more precise tools. For example, a clear increase in the number of vessels was observed, and the skin seems to be thicker. The inventors further showed that fluoxetine and vortioxetine were acting via the stimulation of 5-HT1b receptor.

### 3. Stimulating 5-HT1b receptor involves a pro-regenerative process

Mice were treated with another 5HT1 BR agonist (CP 94253) directly on the wound (topic administration) at a concentration of 0.5µmol, 12h after a wound injury.

No pretreatment was performed on this set of experiments and the drug was administered once a day for the totality of the duration of the wound closure. Controls group received PBS topically only, once a day for the total duration of the treatment.

This direct stimulation of 5HT1 BR triggers a faster regeneration and faster closure of the wound. Indeed 6 days post injury, the wound drops from 15.09±2.3 to 7.7±2.5 (p=0.0019) (figure 5, second column).

Interestingly, the concomitant administration of an antagonist against 5HT1 BR (GR127935 hydrochloride inhibitor), delivered by means of osmotic pumps at 4mg/kg, blocked partially the positive effects observed with the agonist. The size of the wound decreased from 15.09±2.3 to 10.3±3.1 (p=0.01) (figure 5, last column).

These results show that the direct stimulation of 5HT1 BR receptor speeds up the closure of the wound.

The use of 5HT1 BR antagonist showed a partial decrease in this healing effect. This means that 5HT1 BR is sufficient to trigger the pro regenerative effect observed on the skin but that other receptors could be involved as well.

Taken together those results demonstrate that stimulation of 5HT1b is involved in the pro regenerative process but that other mechanisms may be at play.

### REFERENCES

Ahmad W, Faiyaz ul Haque M, Brancolini V, Tsou HC, ul Haque S, Lam H, Aita VM, Owen J, deBlaquiere M, Frank J. et al. (1998). Science; 279: 720-4.

Braiman-Wiksman L, Solomonik I, Spira R, and Tennenbaum T (2007).Toxicologic Pathology; 35:767-779.

Brem H. and Tomic-Canic M. (2007). Cellular and molecular basis of wound healing in diabetes. J. Clin. Invest. 117, 1219-22.

Chabbi-Achengli Y, Coudert AE, Callebert J, Geoffroy V, Côté F, Collet C, de Vernejoul MC (2012) Proc Natl Acad Sci U S A. 2012 Feb 14;109(7):2567-72

Farmaco, 1989, 44, from p.683.

Hinton JM, Hill P, Jeremy J, Garland C. (2000). J Vasc Res. 2000 Nov-Dec;37(6):457-68.

Ito M, Liu Y, Yang Z, Nguyen J, Liang F, Morris RJ and Cotsarelis G (2005). Nat. Med.; 11: 1351-1354.

Kuroda N, Ohyama Y, Nakashima K, Nakashima K, and Akiyama S (1996). Chemical and Pharmaceutical Bulletin; 44(8): 1525-1529.

Levy V, Lindon C, Zheng Y, Harfe BD and Morgan B a (2007). FASEB J.; 21: 1358-1366.

Mokrosz JL, Pietrasiewicz M, Duszynska B, and Cegla M (1992). J. Med. Chem.; 35 (13): 2369-2374.

Müller-Röver S, Handjiski B, Van der Veen C, Eichmüller S, Foitzik K, McKay IA, Stenn KS, and Paus R (2001). J Invest Dermatol.;117(1):3-15.

Nakaya K, Tanaka T, Shirataki Y, Shiozaki H, Funabiki K, Shibata K, Matsui M (2001). Bulletin of the Chemical Society of Japan.; 74(1): 173-177.

Pauwels PJ, Wurch T, Palmier C, Colpaert FC (1998) Br J Pharmacol. 1998 Jan;123(1):51-62.

Reish RG and Eriksson E (2008). Plast. Reconstr. Surg.; 122: 1068-78.

Romanelli MN, Manetti D, Scapecchi S, Borea PA, Dei S, Bartolini A, Ghelardini C, Gualtieri F, Guandalini L, and Varani K (2001). J Med Chem.; 44(23):3946-55.

Sen CK, Gordillo GM, Roy S, Kirsner R, Lambert L, Hunt TK, Gottrup F, Gurtner GC and Longaker MT (2009). Wound Repair Regen; 17: 763-771.

Snippert HJ, Haegebarth A, Kasper M, Jaks V, van Es JH, Barker N, van de Wetering M, van den Born M, Begthel H, Vries RG, et al. (2010). Science (80-.); 327: 1385-1389.

Zhang X, Xu R, Hu X, Luo G, Wu J and Weifeng He Zhang et al. (2015). Burns & Trauma ; 3:15.

## Claims

1. A direct 5-hydroxytryptamine 1B receptor (5-HT1 BR)-stimulating agent for use for boosting the healing, the repairing and/or the treatment of wound, abscess, bedsore, skin ulcer, burn, blister or alopecia in a patient in need thereof, or for use for boosting skin and/or hair repair in patients needing a skin or scalp engraftment.

2. The agent for use according to claim 1, for boosting the healing, the repairing and/or the treatment of skin or scalp after scarification, micro-abrasion or microdermabrasion, micro-incision, microinjection, microperforation, peeling, hair depilation or shaving, electrical stimulation, laser or surgery.

3. The agent for use according to any of claims 1 or 2, wherein said agent is ergotamine or a triptan, said triptan being preferably selected from the group consisting of sumatriptan, rizatriptan, zolmitriptan, eletriptan, almotriptan, frovatriptan, naratriptan, avitriptan, and donitriptan.

4. The agent for use according to any of claims 1 or 2, wherein said agent is vortioxetine, or a derivative thereof, said derivative being vortioxetine which has been modified to comprise at least one charged chemical moiety.

5. The agent for use according to claim 4, wherein said derivative is vortioxetine which has been modified to comprise at least one positively charged chemical moiety.

6. The agent for use according to claim 5, wherein said positively charged chemical moiety is a quaternary ammonium group or a tertiary sulfonium group.

7. The agent for use according to claim 6, wherein
• said quaternary ammonium group has the formula (I)
-(NR₁R₂R₃)⁺Z⁻ (I)
wherein
Z is an organic or inorganic anion; and
R₁, R₂ and R₃ are each independently selected from the group consisting of alkyl, aryl and cycloalkyl;
or
• said tertiary sulfonium group has the formula (II)
-(SR₄R₅)⁺Z⁻ (II)
wherein
Z is an organic or inorganic anion; and
R₄ and R₅ are each independently selected from the group consisting of alkyl, aryl and cycloalkyl.

8. The agent for use according to any of claims 1-4, wherein said agent is a positively charged vortioxetine selected from the group consisting of salts of vortioxetine, vortioxetine coupled to a positively charged amino acid such as histidine, arginine or lysine, pyrrolidinium-vortioxetine, pyperazinium-vortioxetine, dimethylammonium-vortioxetine, sulfonium-vortioxetine, N-oxide-vortioxetine, sulfoxide-vortioxetine, and phosphonium-vortioxetine.

9. The agent for use according to any one of claims 1-8, wherein said alopecia involves inflammation of the scalp, such as scarring alopecia.

10. The agent for use according to any one of claims 1-9, wherein said patients suffer from psychiatric disorders involving self-inflicted skin lesions (SISL).

11. The agent for use according to any one of claim 1 to 10, said agent being administered at the areas receiving a skin, or scalp engraftment prior and/or after the engraftment, or being administered in the graft material before surgery.

12. A direct 5-hydroxytryptamine 1B receptor (5-HT1 BR)-stimulating agent as defined in any one of claims 1 and 3- to 8, combined with a means for inducing skin and/or hair loss, damage or impairment, said means being chosen from: abrasion, injection, peeling, depilation, shaving, or laser, for simultaneous, separate or sequential use for boosting the healing, the repairing and/or the treatment of a wound, abscess, bedsore, skin ulcer, burn, blister or alopecia in a patient in need thereof, or for use as a booster of skin and/or hair repair in a subject needing a skin or scalp engraftment.

## Patentansprüche

1. 5-Hydroxytryptamin-1B-Rezeptor (5-HT1 BR) direkt stimulierendes Mittel zur Verwendung bei der Förderung der Heilung, der Reparatur und/oder der Behandlung einer Wunde, eines Abszesses, eines Dekubitus, eines Hautgeschwürs, einer Verbrennung, einer Blase oder einer Alopezie bei einem Patienten, der diese benötigt, oder zur Verwendung bei der Förderung der Haut- und/oder Haarreparatur bei Patienten, die ein Haut- oder Kopfhautanwachsen benötigen.

2. Mittel zur Verwendung nach Anspruch 1 zur Förderung der Heilung, der Reparatur und/oder der Behandlung von Haut oder Kopfhaut nach Skarifikation, Mikroabrasion oder Mikrodermabrasion, Mikroinzision, Mikroinjektion, Mikroperforation, Peeling, Haardepilation oder Rasur, elektrischer Stimulation, Laser oder Operation.

3. Mittel zur Verwendung nach einem der Ansprüche 1 oder 2, wobei das Mittel Ergotamin oder ein Triptan ist, wobei das Triptan vorzugsweise ausgewählt ist aus der Gruppe, die besteht aus Sumatriptan, Rizatriptan, Zolmitriptan, Eletriptan, Almotriptan, Frovatriptan, Naratriptan, Avitriptan und Donitriptan.

4. Mittel zur Verwendung nach einem der Ansprüche 1 oder 2, wobei das Mittel Vortioxetin oder ein Derivat davon ist, wobei das Derivat Vortioxetin ist, das abgewandelt wurde, um mindestens einen geladenen chemischen Anteil zu umfassen.

5. Mittel zur Verwendung nach Anspruch 4, wobei das Derivat Vortioxetin ist, das abgewandelt wurde, um mindestens einen positiv geladenen chemischen Anteil zu umfassen.

6. Mittel zur Verwendung nach Anspruch 5, wobei der positiv geladene chemische Anteil eine quaternäre Ammoniumgruppe oder eine tertiäre Sulfoniumgruppe ist.

7. Mittel zur Verwendung nach Anspruch 6, wobei
• die quaternäre Ammoniumgruppe die Formel (I) aufweist
-(NR₁R₂R₃)⁺Z⁻ (I)
wobei
Z ein organisches oder anorganisches Anion ist; und
R₁, R₂ und R₃ jeweils unabhängig aus der Gruppe ausgewählt sind, die aus Alkyl, Aryl und Cycloalkyl besteht;
oder
• die tertiäre Sulfoniumgruppe die Formel (II) aufweist
-(SR₄R₅)⁺Z⁻ (II)
wobei
Z ein organisches oder anorganisches Anion ist; und
R₄ und R₅ jeweils unabhängig aus der Gruppe ausgewählt sind, die aus Alkyl, Aryl und Cycloalkyl besteht.

8. Mittel zur Verwendung nach einem der Ansprüche 1 bis 4, wobei das Mittel ein positiv geladenes Vortioxetin ist, das ausgewählt ist aus der Gruppe, die besteht aus Salzen von Vortioxetin, Vortioxetin, das an eine positiv geladene Aminosäure gekoppelt ist, wie beispielsweise Histidin, Arginin oder Lysin, Pyrrolidinium-Vortioxetin, Pyperazinium-Vortioxetin, Dimethylammonium-Vortioxetin, Sulfonium-Vortioxetin, N-Oxid-Vortioxetin, Sulfoxid-Vortioxetin und Phosphonium-Vortioxetin.

9. Mittel zur Verwendung nach einem der Ansprüche 1 bis 8, wobei die Alopezie Entzündung der Kopfhaut, wie beispielsweise vernarbende Alopezie, beinhaltet.

10. Mittel zur Verwendung nach einem der Ansprüche 1 bis 9, wobei die Patienten an psychiatrischen Störungen leiden, die selbst zugefügte Hautläsionen (Self-inflicted Skin lesions - SISL) beinhalten.

11. Mittel zur Verwendung nach einem der Ansprüche 1 bis 10, wobei das Mittel an den Bereichen, die ein Haut- oder Kopfhautanwachsen empfangen, vor und/oder nach dem Anwachsen verabreicht wird oder in dem Transplantatmaterial vor der Operation verabreicht wird.

12. 5-Hydroxytryptamin-1B-Rezeptor (5-HT1 BR) direkt stimulierendes Mittel nach einem der Ansprüche 1 und 3 bis 8, kombiniert mit einem Mittel zum Induzieren von Haut- und/oder Haarverlust, -beschädigung oder - beeinträchtigung, wobei das Mittel ausgewählt ist aus: Abrasion, Injektion, Peeling, Depilation, Rasur oder Laser, zur gleichzeitigen, getrennten oder aufeinanderfolgenden Verwendung zum Fördern der Heilung, der Reparatur und/oder der Behandlung einer Wunde, eines Abszesses, eines Dekubitus, eines Hautgeschwürs, einer Verbrennung, einer Blase oder einer Alopezie bei einem Patienten, der diese benötigt, oder zur Verwendung als ein Mittel zur Förderung der Haut- und/oder Haarreparatur bei einer Zielperson, die ein Haut- oder Kopfhautanwachsen benötigt.

## Revendications

1. Agent stimulant directement le récepteur de 5-hydroxytryptamine 1B (5-HT1 BR) pour une utilisation afin d'amplifier la cicatrisation, la réparation et/ou le traitement d'une plaie, d'un abcès, d'un escarre de décubitus, d'un ulcère cutané, d'une brûlure, d'une ampoule ou d'une alopécie chez un patient en ayant besoin, ou pour une utilisation afin de stimuler la réparation de la peau et/ou des cheveux chez les patients nécessitant une greffe de peau ou de cuir chevelu.

2. Agent pour une utilisation selon la revendication 1, afin d'amplifier la cicatrisation, la réparation et/ou le traitement de la peau ou du cuir chevelu après scarification, micro-abrasion ou microdermabrasion, micro-incision, micro-injection, microperforation, exfoliation, épilation ou rasage des poils, stimulation électrique, laser ou chirurgie.

3. Agent pour une utilisation selon l'une quelconque des revendications 1 et 2, lequel agent est l'ergotamine ou un triptan, ledit triptan étant de préférence choisi dans le groupe constitué par le sumatriptan, le rizatriptan, le zolmitriptan, l'élétriptan, l'almotriptan, le frovatriptan, le naratriptan, l'avitriptan, et le donitriptan.

4. Agent pour une utilisation selon l'une quelconque des revendications 1 et 2, lequel agent est la vortioxétine, ou un dérivé de celle-ci, ledit dérivé étant la vortioxétine qui a été modifiée de manière à comprendre au moins un fragment chimique chargé.

5. Agent pour une utilisation selon la revendication 4, dans lequel ledit dérivé est la vortioxétine qui a été modifiée de manière à comprendre au moins un fragment chimique chargé positivement.

6. Agent pour une utilisation selon la revendication 5, dans lequel ledit fragment chimique chargé positivement est un groupe ammonium quaternaire ou un groupe sulfonium tertiaire.

7. Agent pour une utilisation selon la revendication 6, dans lequel
• ledit groupe ammonium quaternaire répond à la formule (I)
-(NR₁R₂R₃)⁺Z⁻ (I)
dans laquelle
Z est un anion organique ou inorganique ; et
chacun de R₁, R₂ et R₃ est indépendamment choisi dans l'ensemble constitué par alkyle, aryle et cycloalkyle ;
ou
• ledit groupe sulfonium tertiaire répond à la formule (II)
-(SR₄R₅)⁺Z⁻ (II)
dans laquelle
Z est un anion organique ou inorganique ; et
chacun de R₄ et R₅ est indépendamment choisi dans l'ensemble constitué par alkyle, aryle et cycloalkyle.

8. Agent pour une utilisation selon l'une quelconque des revendications 1 à 4, lequel agent est une vortioxétine chargée positivement choisie dans l'ensemble constitué par les sels de vortioxétine, la vortioxétine couplée à un acide aminé chargé positivement tel que l'histidine, l'arginine ou la lysine, la pyrrolidium-vortioxétine, la pipérazinium-vortioxétine, la diméthylammonium-vortioxétine, la sulfonium-vortioxétine, la N-oxyde-vortioxétine, la sulfoxyde-vortioxétine, et la phosphonium-vortioxétine.

9. Agent pour une utilisation selon l'une quelconque des revendications 1 à 8, dans lequel ladite alopécie implique une inflammation du cuir chevelu, telle qu'une alopécie cicatricielle.

10. Agent pour une utilisation selon l'une quelconque des revendications 1 à 9, dans lequel lesdits patients souffrent de troubles psychiatriques impliquant des lésions cutanées volontaires (SISL).

11. Agent pour une utilisation selon l'une quelconque des revendications 1 à 10, ledit agent étant administré au niveau des zones recevant une greffe de peau ou de cuir chevelu avant et/ou après la greffe, ou étant administré dans le matériau de greffe avant la chirurgie.

12. Agent stimulant directement le récepteur de 5-hydroxytryptamine 1B (5-HT1 BR) tel que défini dans l'une quelconque des revendications 1 et 3 à 8, combiné à un moyen pour induire une perte, un dommage ou une détérioration de la peau et/ou des poils, ledit moyen étant choisi parmi : une abrasion, une injection, une exfoliation, une épilation, un rasage ou un laser, pour une utilisation simultanée, séparée ou successive afin d'amplifier la cicatrisation, la réparation et/ou le traitement d'une plaie, d'un abcès, d'un escarre de décubitus, d'un ulcère cutané, d'une brûlure, d'une ampoule ou d'une alopécie chez un patient en ayant besoin, ou pour une utilisation en tant qu'amplificateur de la réparation de la peau et/ou des cheveux chez un sujet nécessitant une greffe de peau ou de cuir chevelu.
